# EUROPEAN PATENT SPECIFICATION

(11) **EP 3 947 410 B1**
(45) Date of publication and mention of the grant of the patent: **12.02.2025**
(21) Application number: 20718527.3
(22) Date of filing: 02.04.2020
(51) Int. Cl.: C07J 41/00, C07J 43/00, A61K 31/58, A61K 31/57, A61K 31/56, A61P 25/00, A61P 25/08

(54) **3ALPHA, 5BETA-NEUROACTIVE STEROIDS FOR THE TREATMENT OF EPILEPSY AND SEIZURE DISEASES**
3ALPHA; 5BETA-NEUROAKTIVE STEROIDE ZUR BEHANDLUNG VON EPILEPSIE UND KRAMPFERKRANKUNGEN
STÉROÏDES 3ALPHA, 5BÊTA-NEUROACTIFS POUR LE TRAITEMENT DE L'ÉPILEPSIE ET DE MALADIES ÉPILEPTIQUES

(30) Priority: 05.04.2019 CZ 20190216
(43) Date of publication of application: 09.02.2022
(73) Proprietor: Ustav organicke chemie a biochemie AV CR, v.v.i., 166 10 Praha 6 (CZ); Fyziologicky ustav AV CR, v.v.i., 14220 Praha 4 (CZ)
(72) Inventor: KUDOVA, Eva, 14800 Praha 4 (CZ); CHODOUNSKA, Hana, 16000 Praha 6 (CZ); MARES, Pavel, 10300 Praha 10 (CZ); VALES, Karel, 15600 Praha 5 (CZ)
(74) Representative: Hartvichova, Katerina
(86) International application number: PCT/CZ2020/050017
(87) International publication number: WO 2020/200335

(56) References cited:
- WO-A2-2009/108804
- CN-A- 108 517 001
- CHRISTOPHER J. MACNEVIN ET AL: "Development and Screening of Water-Soluble Analogues of Progesterone and Allopregnanolone in Models of Brain Injury", JOURNAL OF MEDICINAL CHEMISTRY, vol. 52, no. 19, 8 October 2009 (2009-10-08), pages 6012 - 6023, XP055046453, ISSN: 0022-2623, DOI: 10.1021/jm900712n

## Description

### Field of Invention

The invention is in the field of pharmacy and pharmacology. It relates to compounds with anticonvulsive effect in animal models of epileptic seizures, thus protecting central nervous system (CNS) tissue.

### Background Art

Neurosteroids belong to a family of substances synthesized from cholesterol *de novo* in the brain. Their essential characteristic is the ability to directly influence a range of transmembrane neurotransmitter receptors. From a structural point of view, important factors in the relationship between the chemical structure and the biological activity are geometry between circle A and B (stereochemistry in position C-5), type of substituent in position C-3 and the arrangement of the side chain at C-17. Another important structural factor is the lipophilicity of the molecule (J. Med. Chem. 2015, 58, 5950), which can be synthetically modified. Among the most highly researched neurosteroids belong allopregnanolone (20-oxo-5α-pregnan-3α-ol, ALLO), dehydroepiandrosterone (17-oxo-androst-5-en-3β-ol, DHEA), pregnenolone (20-oxo-pregn-5-en-3β-ol, PREG) a progesterone (pregn-4-en-3,20-dione, PROG). Synthetic analogues of endogenous neurosteroids are called neuroactive steroids. Neuroactive steroids represent molecules with therapeutically interesting properties. They are mostly allosteric receptor modulators of the γ-aminobutyric acid (GABA_{A}) and *N*-methyl-*D*-aspartate acid (NMDA) receptors, the receptors responsible for the CNS excitatory-inhibitory balance. Both neurosteroids and their synthetic analogues (neuroactive steroids) have a range of functions in the central nervous system and periphery from development to management of complex behavior. Current studies show that their effect may lack both psychotomimetic and cognitive side effects (J. Neurosci. 2016, 36, 2161). Neuroactive steroids have undisputed therapeutic potential demonstrated in both *in vitro* and *in vivo* experiments. The neuroprotective effect of neurosteroids or neuroactive steroids is likely mediated at the non-genome level, but it also involves regulating expression of proapoptotic and antiapoptotic factors, engaging signaling cascades in the cell, neurotransmissions, or oxidative and inflammatory processes (Front. Endocrinol. 2011, 2, 50). Anticonvulsant action of neuroactive steroids was demonstrated in animal models of seizures. ALLO and PROG exhibited significant effect in experimental seizures elicited by administration of pentylenetetrazol (Brain Res. 2000, 881, 98), pilocarpine (Neuropharmacology 1996, 35, 1049), NMDA (J. Pharmacol. Exp. Ther. 1997, 282, 543) and kainic acid (Psychoneuroendocrinology 2000, 25, 407). As ALLO potentiates GABA_{A} receptors it deceases frequency of seizures, mortality and cellular death in animal models of epilepsy (Pol. J. Pharmacol. 1997, 49, 411). A synthetic derivative of ALLO Ganaxolone was able to prevent generation of seizures in rodents (Epilepsia 2010, 51, 84). Some synthetic steroids like ganaxolone, alfaxalone, hydroxydione and minaxolone were tested as sedatives and anesthetics in clinical studies. The most successful synthetic steroid is ganaxolone, C-3 methylated derivative of ALLO developed by Marinus Pharmaceutical. It is positive allosteric modulator of GABA_{A} receptors efficient as an antiepileptic in many animal models. Clinical studies demonstrated good tolerability in adults as well as children. Epilepsies are characterized by more than 25 syndromes with various types of seizures which differ in severity as well as in response to therapy (Mental Health Clinician 2017,7, 235). Patients with epilepsies might exhibit also different psychiatric symptoms (e.g. cognitive and behavioral changes) complicating often treatment of epileptic syndromes (Expert Opin. Drug Saf. 2011, 10, 913).

Common psychic comorbidities associated with epilepsy include depression, anxiety, attention deficit disorders and psychosis in prevalence ranging from 20% to 30% (Expert Opin. Drug Saf. 2011, 10, 913). Depression is one of the most common psychiatric comorbidities in epilepsy patients with a prevalence ranging from 20% to 55%, but in some populations, the prevalence may reach up to 80% (Expert Opin. Drug Saf. 2011, 10, 913). According to a number of authors, anxiety disorders are the second most common psychiatric comorbidity in epilepsy patients immediately after a depressive disorder, while others report even a more frequent incidence of anxiety disorders. As it has been repeatedly shown that there is a significant relationship between the incidence of anxiety disorders and the quality of life of epilepsy patients, appropriate therapy for anxiety and anxiety disorders in epileptological practice should therefore be one of the priorities of caring for these individuals. This approach has already been experimentally verified for neurosteroids. There are several animal and human studies suggesting that PROG, DHEA and PREG are involved in the mechanism of action of antidepressants (Neuroscience 2011, 191, 55).

The neuroprotective effect of steroid derivatives with a substituent capable of forming ion at position C-3 is claimed by patents US 8575376, EP 2435463 and US 15/506318. These documents claim pregnane derivatives (polar acetyl substituent in position C-17) and androstane derivatives (non-polar substituent in position C-17) substituted in position C-3 by ionisable substituent, respectively. The neuroprotective effect of these charged derivatives is directly linked to the specific combination of structural features at the C-3 (3α-charged substituent) and C-5β positions, and to the ability of these substances to inhibit ionotropic glutamate NMDA receptors. Alongside, current articles and patent literature claim that NMDA receptor inhibition, hence the neuroprotective effect is contingent on a charged C-3 substituent, and uncharged analogues lack a biological effect on NMDA receptor modulation (J. Pharmacol. Exp. Ther. 2000, 293, 747; Mol. Pharmacol. 1997, 52, 1113). The derivatives claimed in the invention application submitted here have a cyclic or acyclic C-3 substituent that does not carry the charge.

Current scientific and patent literature (US 9,527,881; J. Med. Chem. 2009, 52, 6012) lists the neuroprotective effect of C-3 substituted steroid analogues of PROG bearing proline or acyclic substituent with the NH₂ group. These substances are seemingly structurally similar to those claimed in the application attached here. Specifically, e.g. 3β-L-proline-progesterone-HCl (PI-33) of the above-mentioned patent. The substances are described as steroid analogues for prevention and treatment of neurodegeneration in patients with central nervous system injury. Substance PI-33 was resynthesized and tested in a model of pentylenetetrazol-induced seizures. Administration of 1 and 10 mg/kg doses did not change incidence of generalized tonic-clonic seizures. All animals with either dose exhibited this type of seizures. It might be concluded that anticonvulsant action of substances with cyclic or acyclic substituent with NH or NH₂ group cannot be generally predicted and substitution or modification at C-3 position is always unique and it is impossible to propose a structure in an additive way.

The neuroprotective effect of steroid derivatives is also claimed by the application of US 20170246188 A1 (Method of Treating organophosphate intoxication) with the presumed anticonvulsant effect. The claims of this application can be considered speculative as the neurosteroid substances claimed are defined as pregnanes, androstanes, 19-norandrostanes and 19-norpregnanes substituted by groups of the general formula that define no type or position of substituent on the steroid skeleton. Such defined general formula generally covers hundreds to thousands of endogenous or synthetic substances. As an example, we can name bile acids, which are a component of bile and play a major role in the digestion of lipids, in the metabolism of cholesterol and its removal from the body, or the endogenous glucocorticoid hormone tetrahydrocorticosterone could be mentioned as an additional example. While the above randomly named substances meet the definition of the general formula, a person skilled in the art is clearly able to estimate in advance that these substances are ineffective in treating the organophosphate intoxication with consequences defined as so-called cholinergic crisis symptoms. Its effect was also tested in a model of pentylenetetrazol-elicited seizures in rats. Allopregnanolone (S)-5-oxopyrrolidin-2-carboxylate has only a moderate action against generalized tonic-clonic seizures induced by a high subcutaneous dose of pentylenetetrazol (100 mg/kg) in 12- as well as 25-day-old rats. These results clearly show that the therapeutic effect of neurosteroids cannot be predicted in general and that substitution or modification to C-3, possibly a D-ring in combination with the size and type of the substituent in the C-3 position, is always quite unique, impossible to predict in advance and impossible to design with an additive approach.

### Disclosure of the Invention

Compounds according to the present invention exhibit markedly high efficiency in P12 rats, *i.e.* in the period corresponding to early postnatal period of human babies. The compounds of the present invention are selectively active against age-dependent epileptic syndromes. Epileptic syndromes and epilepsies in immature brain possess biological parameters markedly different from those in mature brain. High seizure susceptibility and easy generation of epileptic seizures is due to postnatal development of brain which continues at least up to prepubertal period. Inhibitory mechanisms are not fully developed and stabilized at early developmental stages and there is an intense development of neuronal networks including changes of neurotransmitters and neuromodulators. Epilepsies in early developmental stages develop on a neurobiological substrate of constantly changing and developing brain, in contrast to epilepsies in adults. Pediatric epileptology describes specific age-bound epileptic syndromes present only in infancy, childhood and adolescence. In addition, there are also epilepsies identical with those present in adulthood, *i.e.* syndromes which are not age-bound. This results in a much broader spectrum of childhood epilepsies than adult epilepsies.

Exact diagnosis is decisive for adequate pharmacotherapy. Some of the age-bound (age-specific, age-dependent) epilepsies are insensitive to clinically used antiepileptic drugs - it is a reason for development of a specific pharmacologic category of age-specific antiepileptic drugs. Type of epilepsy is important for prognosis and brain development. There are benign epileptic syndromes only minimally affecting behavioral symptoms and neuropsychological parameters. On the other hand, there are serious epileptic syndromes, usually in connection with encephalopathy. These epilepsies are classified as catastrophic because they result in a cognitive deficit and possible reversion of psychomotoric development. There is only a minimal chance for full compensation. It is the age-specific efficacy of the compounds of the invention which determines their use in age-bound diagnoses (including rare diseases). The compounds of the invention will be important in cases of presence of both psychiatric and neurodevelopmental syndromes and comorbidities during ontogeny as well as in adulthood. The compounds of the present invention exhibit promising results in animal models of psychiatric diseases in developing and adult rats.

Age-specific efficacy has not yet been observed for any known neuroactive steroids with a structure relatively close to the compounds of the present invention. Therefore, the claimed compounds are specific and unique in their pharmacological profile. Their biological effects cannot be derived from the available state of the art.

In the assays performed by the inventors, doses of 1, 5, and 10 mg/kg of the compounds of the present invention significantly suppressed convulsive seizures. The highest dose (10 mg/kg *i.p.*) abolished pentylenetetrazol-induced seizures in immature animals and significantly decreased incidence and prolonged latency to generalized tonic-clonic seizures in adult rats. The 10-mg/kg dose nearly completely blocked seizures elicited by the 60-mA stimulation current intensity.

The present invention provides compounds of general formula I, 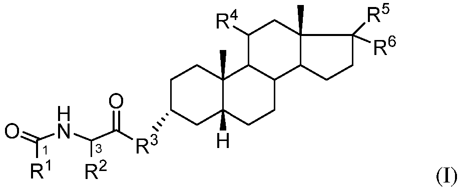 wherein,
- R¹ represents methyl, and then R² is a hydrogen atom or linear or branched C₁-C₄ alkyl,
   or
   R¹ and R² form together a group -(CH₂)ₚ-, wherein p=2 or 3, which forms five- or six-membered ring together with carbon atoms 1 and 3 and the nitrogen atom of the general formula I,
- R³ represents -O-(CH₂)ₙ-Oₘ-, where n = 0, 1, or 2 and m = 0 or 1,
- R⁴ is a hydrogen atom or hydroxyl group in position C-11 of steroidal skeleton,
- R⁵ is a hydrogen atom, and then R⁶ is substituent at position C-17 of steroidal skeleton selected from a group consisting of a hydrogen atom, acetyl group, cyano group, C₁-C₂ cyanoalkyl group, 1,1-difluoroethyl group and linear or branched C₁- C₄ alkyl,
   or R⁵ and R⁶ together form a structure selected from a group consisting of C₁-C₂ alkylidene, cyanomethylene group, atom of oxygen, two atoms of fluorine.

Group definitions are used as generally understood by a person skilled in the art.

Alkyl is a radical of linear or branched C₁ to C₄, preferably C₁ to C₃, most preferably C₁ to C₂ saturated aliphatic hydrocarbon chain, formed by the removal of one hydrogen atom.

Alkylene is a linear, branched or cyclic, preferably linear or branched, divalent aliphatic hydrocarbon chain.

Acetyl group means the -CO-CH₃ group.

Cyano group means -C=N.

Cyanoalkyl group means a group consisting of an alkyl group as defined above with one hydrogen atom replaced by a -C=N group.

Cyanomethylene group means the group =CH-C=N.

1,1-difluoroethyl group is -CF₂-CH₃ group.

C₁-C₂ Alkylidene is a divalent aliphatic hydrocarbon chain, containing a double bond (=CH₂ or =CH-CH₃).

The term "hydroxyl" refers to -OH group.

In a preferred embodiment, the present invention provides the following compounds of formula I:
(3R,5R,8R,9S,10S,13S,14S,17S)-17-Acetyl-10,13-dimethylhexadecahydro-1H-cyclopenta[a]fenantren-3-yl 5-oxopyrrolidine-2-carboxylate (1),
(3R,5R,8R,9S,10S,13S,14S,17S)-17-Acetyl-10,13-dimethylhexadecahydro-1H-cyclopenta[a]phenantren-3-yl 6-oxopiperidine-2-carboxylate (2)
(3R,5R,8R,9S,10S,13S,14S,17S)-17-Cyano-10,13-dimethylhexadecahydro-1H-cyclopenta[a]phenanthren-3-yl 5-oxopyrrolidine-2-carboxylate (3),
(3R,5R,8S,9S,10S,11R,13S,14S,17S)-17-Acetyl-11-hydroxy-10,13-dimethylhexadecahydro-1H-cyclopenta[a]phenanthren-3-yl 5-oxopyrrolidine-2-carboxylate (4),
(3R,5R,8S,9S,10S,13S,14S)-10,13-Dimethylhexadecahydro-1H-cyclopenta[a]phenanthren-3-yl 5-oxopyrrolidine-2-carboxylate (5),
(3R,5R,8R,9S,10S,13S,14S)-10,13-Dimethyl-17-oxohexadecahydro-1H-cyclopenta[a]phenanthren-3-yl 5-oxopyrrolidine-2-carboxylate (6),
(3R,5R,8S,9S,10S,13S,14S)-10,13-Dimethylhexadecahydro-1H-cyclopenta[a]phenanthren-3-yl 6-oxopiperidine-2-carboxylate (7),
(3R,5R,8R,9S,10S,13S,14S,17S)-17-Cyano-10,13-dimethylhexadecahydro-1H-cyclopenta[a]phenanthren-3-yl 6-oxopiperidine-2-carboxylate (8),
(3R,5R,8R,9S,10S,13S,14S,17S)-17-Acetyl-10,13-dimethylhexadecahydro-1H-cyclopenta[a]phenanthren-3-yl acetylglycinate (9),
(3R,5R,8R,9S,10S,13S,14S,17S)-17-Acetyl-10,13-dimethylhexadecahydro-1H-cyclopenta[a]phenanthren-3-yl acetylleucinate (10),
(3R,5R,8S,9S,10S,13R,14S,17S)-10,13,17-Trimethylhexadecahydro-1H-cyclopenta[a]phenanthren-3-yl 5-oxopyrrolidine-2-carboxylate (11),
(3R,5R,8R,9S,10S,13S,14S,Z)-17-Ethylidene-10,13-dimethylhexadecahydro-1H-cyclopenta[a]phenanthren-3-yl 5-oxopyrrolidine-2-carboxylate (12),
2-(((3R,5R,8S,9S,10S,13S,14S)-10,13-Dimethylhexadecahydro-1H-cyclopenta[a]phenanthren-3-yl)oxy)ethyl 5-oxopyrrolidine-2-carboxylate (13),
2-(((3R,5R,8S,9S,10S,13S,14S)-10,13-Dimethylhexadecahydro-1H-cyclopenta[a]phenanthren-3-yl)oxy)ethyl 6-oxopiperidine-2-carboxylate (14),
2-((3R,5R,8R,9S,10S,13S,14S,17S)-17-Acetyl-10,13-dimethylhexadecahydro-1H-cyclopenta[a]phenanthren-3-yl)ethyl 5-oxopyrrolidine-2-carboxylate (16),
2-((3R,5R,8R,9S,10S,13S,14S,17S)-17-Acetyl-10,13-dimethylhexadecahydro-1H-cyclopenta[a]phenanthren-3-yl)ethyl 6-oxopiperidine-2-carboxylate (17).

Another object of the invention are the compounds of general formula I and the corresponding specific compounds listed herein above, for use as medicaments.

An aspect of the invention are the compounds of general formula I for use in treating epilepsy or conditions associated with convulsions, such as seizures associated with hypoxia; seizures associated with traumatic brain damage; seizures associated with intoxication; pathological changes caused by hyperexcitation.

As aspect of the invention are the compounds of general formula I for use in treating conditions that may accompany epilepsy, such as affective disorders, depression, post-traumatic stress disorder (PTSD) and stress-related diseases, anxiety, schizophrenia and psychotic disorders, related ischemic CNS damage, neurodegenerative changes and disorders, multiple sclerosis.

The invention also includes the use of compounds of general formula I for the manufacture of a veterinary or human medicament for the treatment of epilepsy and comorbidities associated with it or other conditions associated with convulsions, such as seizures associated with hypoxia; seizures associated with traumatic brain damage; seizures associated with intoxication; pathological changes caused by hyperexcitation; or for the treatment of conditions that may accompany epilepsy, such as affective disorders, depression, post-traumatic stress disorder (PTSD) and stress-related diseases, anxiety, schizophrenia and psychotic disorders, related ischemic CNS damage, neurodegenerative changes and disorders, multiple sclerosis.

In an aspect, the invention further includes the compounds of the present invention for use in a method of treatment of epilepsy and comorbidities associated with it or other conditions associated with convulsions, such as seizures associated with hypoxia; seizures associated with traumatic brain damage; seizures associated with intoxication; pathological changes caused by hyperexcitation; or for the treatment of conditions that may accompany epilepsy, such as affective disorders, depression, post-traumatic stress disorder (PTSD) and stress-related diseases, anxiety, schizophrenia and psychotic disorders, related ischemic CNS damage, neurodegenerative changes and disorders, multiple sclerosis, said method comprising the step of administering at least one compound of general formula I to a patient in need of such treatment.

The object of the invention is also a pharmaceutical composition, which contains, as an active ingredient, at least one compound of the general formula I. The compound of general formula I may preferably be selected from the list of specific preferred compounds provided herein above.

The pharmaceutical composition may be suitable or destined for human or veterinary use. Pharmaceutical compositions typically further contain pharmaceutically acceptable excipients, such as fillers, binders, solvents, diluents, glidants, lubricants, stabilizers, preservatives, colorants, coatings, etc. Suitable excipients and their use are known to a person skilled in the art of pharmaceutical formulation. The object of the invention is also the aforementioned pharmaceutical composition for use in the treatment of epilepsy and comorbidities associated with it or other conditions associated with convulsions, such as seizures associated with hypoxia; seizures associated with traumatic brain damage; seizures associated with intoxication; pathological changes caused by hyperexcitation.

The object of the invention is also the aforementioned pharmaceutical composition for use in the treatment of conditions that may accompany epilepsy, such as affective disorders, depression, post-traumatic stress disorders (PTSD) and stress-related diseases, anxiety, schizophrenia and psychotic disorders, associated ischemic CNS damage, neurodegenerative changes and disorders, multiple sclerosis.

The invention also includes the compounds of general formula I for use as active ingredients or auxiliary ingredients contained in food supplements or cosmetic products destined to improve responses of individual parts of the body to convulsion-related diseases.

### Brief description of the drawings

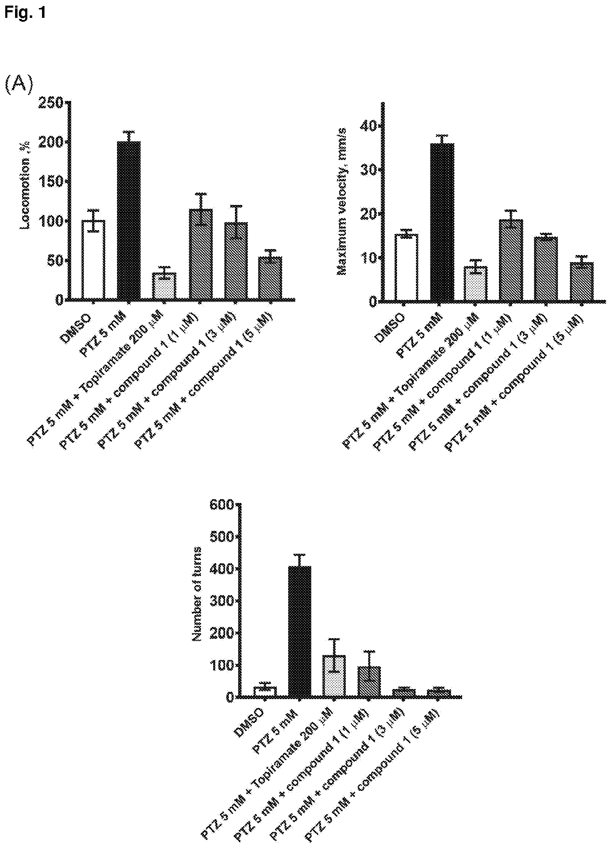

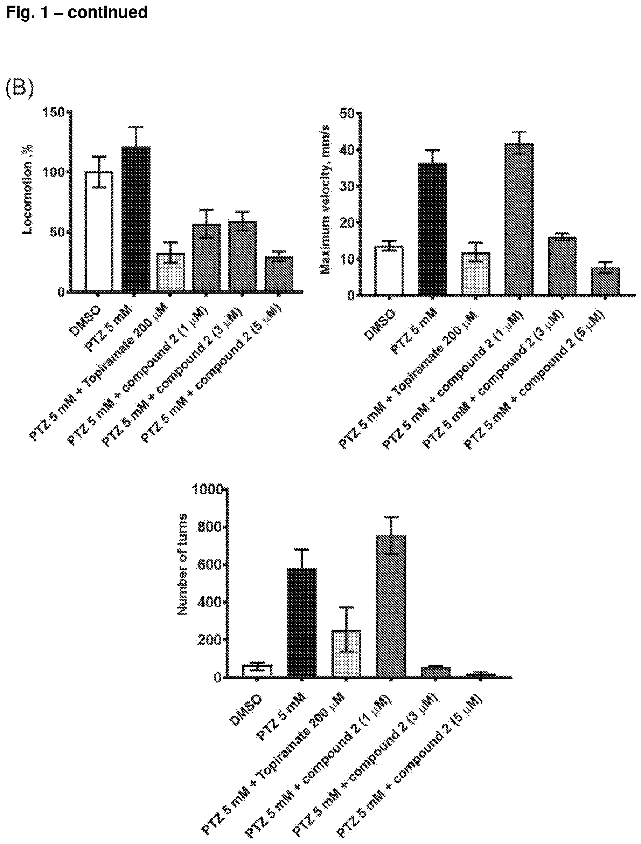

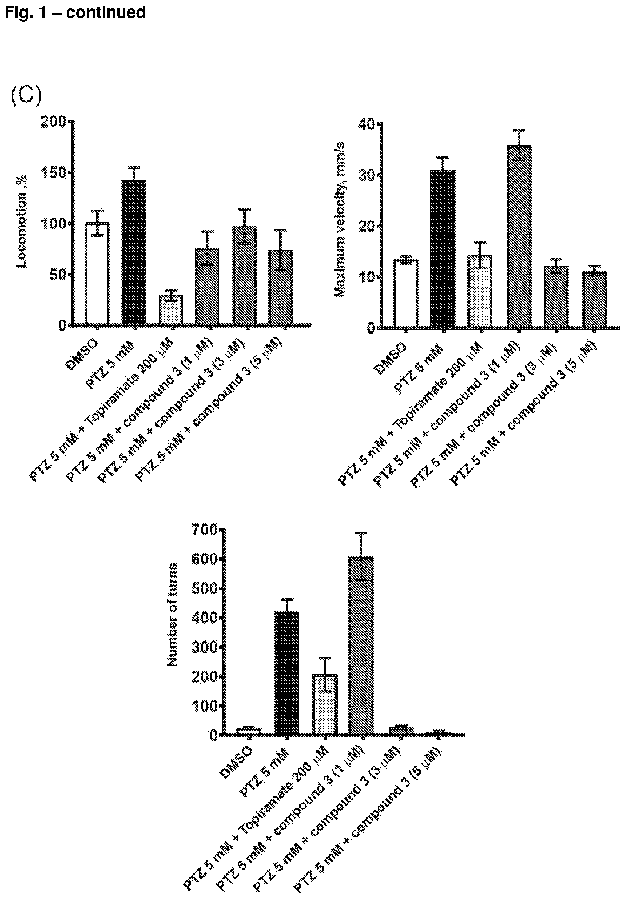

**Fig 1****.** shows anticonvulsant effect of substances from Example 1, 2, and 3 in a model of PTZ-induced seizures in zebrafish *(Danio rerio).* Substances from Example 1 (A), 2 (B) and 3 (C) were used in doses of 1, 3, and 5 mg/kg. Individual graphs demonstrate an effect on spontaneous locomotor activity (left graph), swimming velocity (middle graph) and number of turns (specific unpredictable movements, right graph).

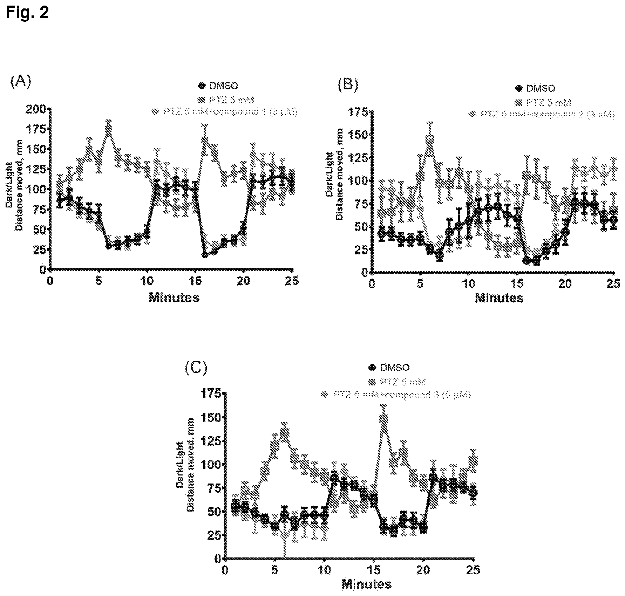

**Fig 2****.** shows anticonvulsant effect of substances from Example 1, 2, and 3 in a model of PTZ-induced seizures in zebrafish *(Danio rerio).* Substances from Example 1 (A), 2 (B) and 3 (C) were used in doses of 1, 3, and 5 mg/kg. Individual graphs demonstrate an effect on spontaneous locomotor activity and dark/light behavioral patterns.

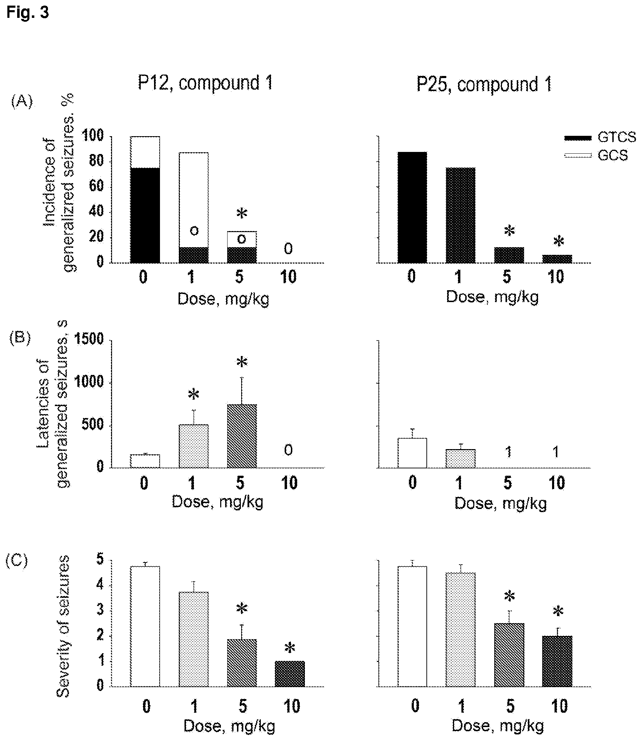

**Fig 3****.** shows anticonvulsant effect of a substance from Example 1 in a model of PTZ-induced seizures in 12- and 25-day-old rats. Doses of 1, 5, and 10 mg/kg *i.p.* were used (x-axis). Graph A (y-axis in %) - incidence of generalized seizures with a tonic phase (GTCS) and incomplete seizures without the tonic phase (GCS); graph B (y-axis in s) - latencies to onset of generalized seizures; graph C (y-axis is score) - severity of seizures evaluated by means of a 5-point scale; each animal was classified according to the most severe event. Scale: 0 - no activity; 1 - isolated myoclonic jerks; 2 - isolated elements of minimal clonic seizures and/or epileptic automatisms; 3 - minimal clonic seizures with preserved righting ability; 4 - incomplete generalized seizures (GCS); 5 - complete generalized tonic-clonic seizures (GTCS). Abbreviations: P12 - 12-days old animals; P25 - 25-days old animals. Asterisks denote a significant difference in comparison with controls; 0 or 1 - seizures were abolished or present in one rat only.

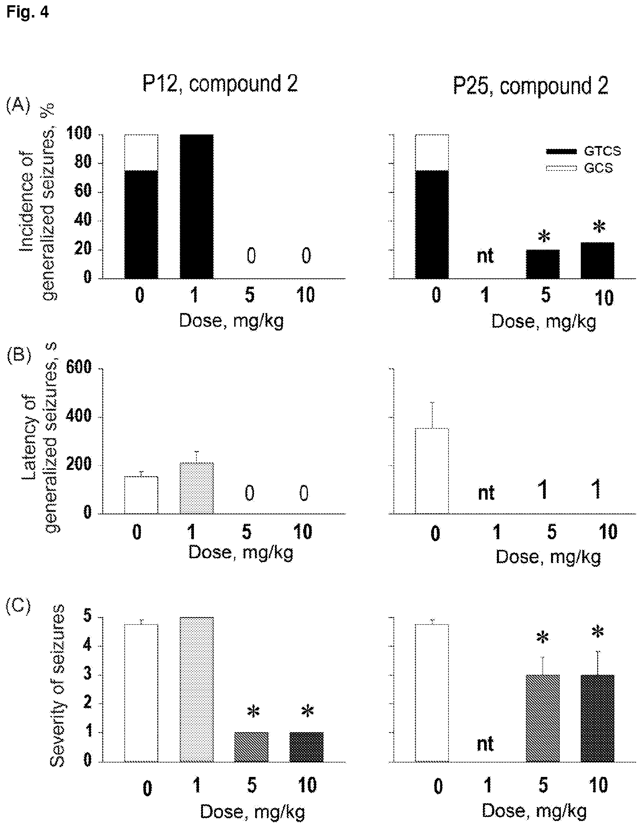

**Fig 4****.** shows anticonvulsant effect of a substance from Example 2 in a model of PTZ-induced seizures in 12- and 25-day-old rats. Doses of 1, 5, and 10 mg/kg *i.p.* were used (x-axis). Graph A (y-axis in %) - incidence of generalized seizures with a tonic phase (GTCS) and incomplete seizures without the tonic phase (GCS); graph B (y-axis in s) - latencies to onset of generalized seizures; graph C (y-axis is score) - severity of seizures evaluated by means of a 5-point scale; each animal was classified according to the most severe event. Scale: 0 - no activity; 1 - isolated myoclonic jerks; 2 - isolated elements of minimal clonic seizures and/or epileptic automatisms; 3 - minimal clonic seizures with preserved righting ability; 4 - incomplete generalized seizures (GCS); 5 - complete generalized tonic-clonic seizures (GTCS). Abbreviations: P12 - 12-days old animals; P25 - 25-days old animals. Asterisks denote a significant difference in comparison with controls; 0 or 1 - seizures were abolished or present in one rat only; nt - not tested.

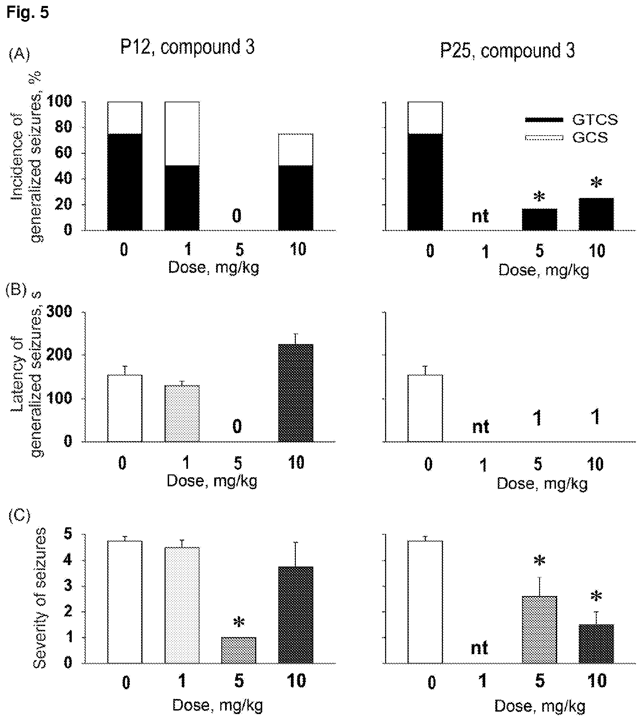

**Fig 5****.** shows anticonvulsant effect of a substance from Example 3 in a model of PTZ-induced seizures in 12- and 25-day-old rats. Doses of 1, 5, and 10 mg/kg *i.p.* were used (x-axis). Graph A (y-axis in %) - incidence of generalized seizures with a tonic phase (GTCS) and incomplete seizures without the tonic phase (GCS); graph B (y-axis in s) - latencies to onset of generalized seizures; graph C (y-axis is score) - severity of seizures evaluated by means of a 5-point scale; each animal was classified according to the most severe event. Scale: 0 - no activity; 1 - isolated myoclonic jerks; 2 - isolated elements of minimal clonic seizures and/or epileptic automatisms; 3 - minimal clonic seizures with preserved righting ability; 4 - incomplete generalized seizures (GCS); 5 - complete generalized tonic-clonic seizures (GTCS). Abbreviations: P12 - 12-days old animals; P25 - 25-days old animals. Asterisks denote a significant difference in comparison with controls; 0 or 1 - seizures were abolished or present in one rat only; nt - not tested.

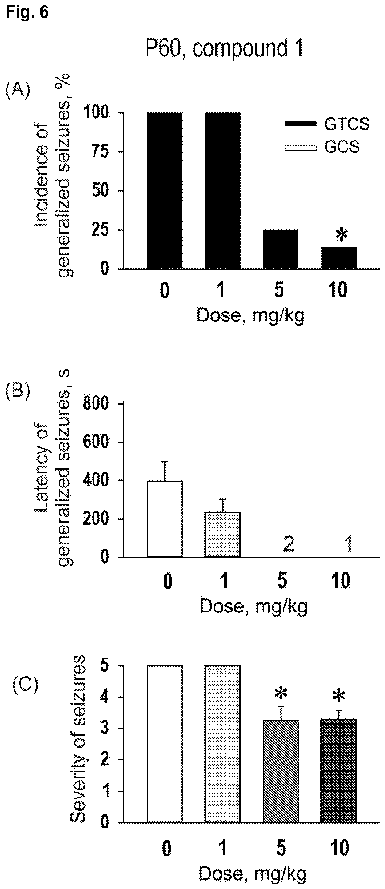

**Fig. 6****.** shows anticonvulsant effect of a substance from Example 1 in a model of seizures elicited by 6 Hz transcorneal electrical stimulation in young adult (P60) male rats. Steroid substance in dose 1, 5, or 10 mg/kg *i.p.* (x-axis) was administered 20 min before the first stimulation. Graph A (y-axis in %) - incidence of complete (GTCS) and incomplete, *i.e.* without the tonic phase (GCS) generalized seizures; graph B (y-axis in %) - incidence of minimal clonic seizures; graph C (y-axis in s) - latencies of generalized seizures; graph D (y-axis in s) - latencies of minimal clonic seizures; graph E (y-axis is score) - seizure severity evaluated by means of a 5-point scale; each animal was classified according to the most severe event. Scale: 0 - no activity; 1 - isolated myoclonic jerks; 2 - isolated elements of minimal clonic seizures and/or epileptic automatisms; 3 - minimal clonic seizures with preserved righting ability; 4 - incomplete generalized seizures (GCS); 5 - complete generalized tonic-clonic seizures (GTCS). Asterisks denote a significant difference in comparison with controls; 1 or 2 - seizures were abolished or present in one or 2 rats only.

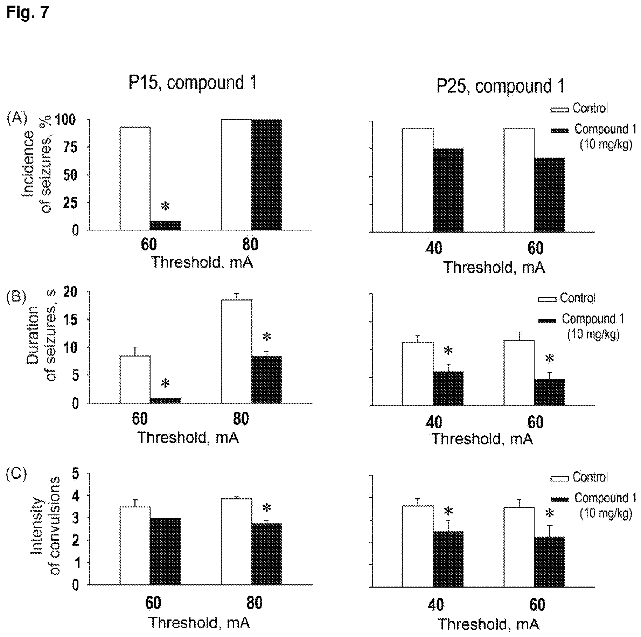

**Fig. 7****.** shows anticonvulsant effect of a substance from Example 1 in a model of seizures elicited by 6 Hz transcorneal electrical stimulation in 15- and 25-day-old male rats. X-axis - current intensities used (60 and 80 mA in P15 and 40 and 60 mA in P25 rats). Y-axis - incidence of seizures in % (A); duration of seizures in s (B), and seizure severity expressed as a score (C). Seizure severity evaluated by means of a 5-point scale; each animal was classified according to the most severe event. Scale: 0 - no activity; 1 - isolated myoclonic jerks; 2 - isolated elements of minimal clonic seizures and/or epileptic automatisms; 3 - minimal clonic seizures with preserved righting ability; 4 - incomplete generalized seizures (GCS); 5 - complete generalized tonic-clonic seizures (GTCS). Asterisks denote a significant difference in comparison with controls. Abbreviations: P12 - 12-days old animals; P25 - 25-days old animals.

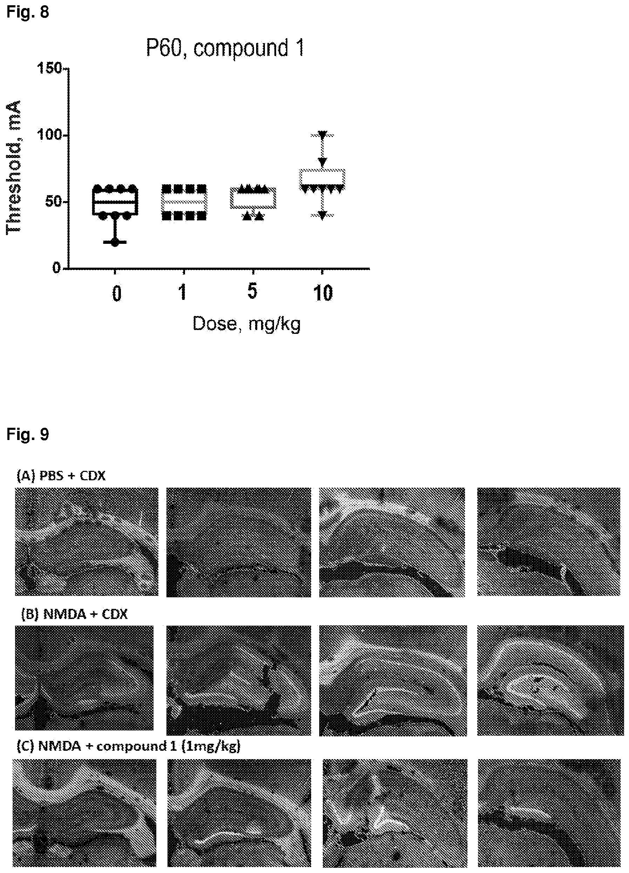

**Fig. 8****.** shows anticonvulsant effect of a substance from Example 1 in a model of seizures elicited by 6 Hz transcorneal electrical stimulation in young adult (P60) male rats presented as box plots (median with 25 and 75%) and maximal and minimal values (y-axis); doses in mg/kg (x-axis). Symbols in individual boxes show individual values for animals.
**Figure 9****.** Histological damage, respectively neuroprotective effect of Compound from Example 1 at dose 1 mg/kg in the model of NMDA lesion, a model of the glutamate-induced excitotoxicity. The tissue damage is induced by infusing NMDA (25 mmol.L⁻¹) into the dorsal hippocampus in the male rats. The figures in panel (A) show representative images of brain slices of control animals, in panel (B) the group of animals which were administered the solutions containing NMDA into dorsal hippocampus (NMDA lesion) and *i.p.* treated by (2-hydroxypropyl)-β-cyclodextrin (CDX) and in panel (C) brain slices of the animals, with NMDA lesions of the dorsal hippocampus and *i.p.* treated by the Compound of Example 1 at a dose of 1 mg / kg dissolved in CDX.

The present invention will be further illustrated by Examples, which should not be construed as limiting the scope of the invention.

### List of Abbreviations:

- CHCl₃: chloroform
- CDX: (2-hydroxypropyl)-β-cyclodextrin
- DCM: dichloromethane
- DMAP: 4-dimethylaminopyridine (IUPAC: *N*,*N*-Dimethylpyridin-4-amine)
- DMF: dimethylformamide (IUPAC: *N*,*N*-Dimethylformamide)
- DMSO: dimethyl sulfoxide
- EDCI: 1-ethyl-3-(3-dimethylaminopropyl)carbodiimide hydrochloride
- ESI: electrospray ionization
- HPLC: high-performance liquid chromatography)
- HRMS: high resolution mass spectrometry
- IČ: infrared spectroscopy
- MS: mass spectrometry
- NMDA: N-methyl-D-aspartic acid
- NMR: nuclear magnetic resonance
- PBS: phosphate buffered saline

### Experimental Part - Chemistry

The reactions which require anhydrous conditions were always carried out in a pre-dried apparatus and under an inert atmosphere. Samples for analysis were dried over phosphorous pentoxide at 50 °C and a pressure of 100 mbar. Solvents were removed from the solution by rotary evaporator (0.25 kPa) at 50 °C bath. Thin layer chromatography (TLC) was performed on plates coated with a thin layer of silica gel (ICN Biochemicals). Preparative column chromatography was performed on silica gel Fluka (60 microns). Melting points were measured at Hund Wetzlar H-600 (Helmut Hund, Germany). Optical rotation was measured in chloroform Autopol IV polarimeter (Rudolph Research Analytical, Flanders, USA), [α]_{D} values are shown in [10⁻¹.deg.cm².g⁻¹], concentration values as [g·100 ml⁻¹] and were compensated to a standard temperature of 20° C. Infrared spectra were measured in chloroform using a Nicolet 6700 (Thermo Scientific, USA). NMR spectra were measured in FT mode on Bruker AVANCE III^{™} 400 MHz with tetramethylsilane (TMS) as internal standard. Chemical shifts are given in ppm (δ-scale), coupling constants (J) are given in Hz. Signal multiplicities are designated as follows: s - singlet, d - doublet, t - triplet, q - quartet, m - multiplet, br denotes broad. Mass spectra were measured on a LTQ Advantage Thermo spectrometer with ESI or EI ionization (10 eV) in positive or negative mode.

### General Procedures

### General Procedure A

A mixture of 3-hydroxy steroid (1.0 mmol), 4-dimethylaminopyridine (DMAP, 0.25 mmol), 1-ethyl-3-(3-dimethylaminopropyl)carbodiimide hydrochloride (EDCI, 2.7 mmol), and hydroxybenzotriazole (HOBt (2.2 mmol) was dried under vacuum at room temperature for 1 hour. Then, dry dimethylformamide (10 mL) was added under inert atmosphere, followed by slow dropwise addition of particular carboxylic acid (1.5 mmol) in dry DMF (5 mL). The reaction mixture was allowed to stir overnight at room temperature. After solvent evaporation, the residue was purified by a column chromatography.

### General Procedure B

A mixture of 3-hydroxy steroid (1.0 mmol), 4-dimethylaminopyridine (DMAP, 0.25 mmol), and particular carboxylic acid (1.5 mmol) was dried under vacuum at room temperature for 1 hour. Then, dry dichloromethane (DCM, 2 mL) was added under inert atmosphere, followed by slow dropwise addition of 1-ethyl-3-(3-dimethylaminopropyl)carbodiimide hydrochloride (EDCI, 2.7 mmol) in dry DCM (10 mL). The reaction mixture was allowed to stir overnight at room temperature. After 18 h, it was poured into water. The combined extracts were washed with aqueous HCl (5%), aqueous NaHCO₃, brine, and dried over anhydrous Na₂SO₄. After solvent evaporation, the residue was purified column chromatography on silica gel.

### Example 1: (3R,5R,8R,9S,10S,13S,14S,17S)-17-Acetyl-10,13-dimethylhexadecahydro-1H-cyclopenta[a]fenantren-3-yl 5-oxopyrrolidine-2-carboxylate (1)

Compound 1 was prepared according to the General Procedure A (DMF). Starting from 20-oxo-5β-pregnan-3α-ol (318 mg, 1.0 mmol), using *L*-pyroglutamic acid (194 mg, 1.5 mmol), compound 1 (370 mg, 89%) was obtained by column chromatography on silica gel (5% acetone/chloroform): mp 167-168 °C (chloroform, diethyl ether), [α]_{D}²⁰ +105.6 (c 0.3, CHCl₃). ¹H NMR (400 MHz, CDCl₃): δ 0.60 (3H, s, H-18), 0.94 (3H, s, H-19), 2.11 (3H, s, H-21), 4.16-4.24 (1H, m, H-C2'), 4.80 (1H tt, J= 11.4, 4.8 Hz, H-3), 5.86 (1H, s, N-H). ¹³C NMR (101 MHz, CDCl₃): δ 209.7, 177.6, 171.5, 75.9, 64.0, 56.8, 55.6, 44.5, 41.9, 40.6, 39.3, 35.9, 35.1, 34.7, 32.2, 31.7, 29.3, 26.9, 26.7, 26.4, 25.1, 24.6, 23.4, 23.1, 21.0, 13.6. IR spectrum (CHCl₃): 1734, 1702 (C = O), 1230 (C-O). MS: ESI m/z 452.3 (100%, M+Na). HR-MS (ESI) m/z: for C₂₆H₃₉NO₄Na [M+Na] calcd, 452.27713; found, 452.26742. For C₂₆H₃₉NO₄ (429.6) calcd: 72.69%, C; 9,15%, H; 3.26%, N. Found: 72.29%, C; 9,15%, H; 3.11%, N.

### Example 2: (3R,5R,8R,9S,10S,13S,14S,17S)-17-Acetyl-10,13-dimethylhexadecahydro-1H-cyclopenta[a]phenantren-3-yl 6-oxopiperidine-2-carboxylate (2)

Compound 2 was prepared according to the General Procedure B (DCM). Starting from 20-oxo-5β-pregnan-3α-ol (318 mg, 1.0 mmol), using 6-oxo-L-pipecolic acid (213 mg, 1.5 mmol), compound 2 (142 mg, 32%) was obtained by column chromatography on silica gel (3% acetone/chloroform): mp 139-141 °C (acetone/n-heptane), [α]_{D}²⁰ +103.2 (c 0.3, CHCl₃). ¹H NMR (400 MHz, CDCl₃): δ 0.60 (3H, s, H-18), 0.94 (3H, s, H-19), 4.05 (1H, m, H-C2'), 4.81 (1H, m, H-3), 6.14 (1H, s, N-H). ¹³C NMR (101 MHz, CDCl₃): δ 209.7, 171.4, 170.5, 76.1, 64.0, 56.8, 55.1, 44.5, 42.0, 41.0, 39.3, 35.9, 35.0, 34.7, 32.2, 31.7, 31.2, 27.0, 26.7, 26.4, 25.6, 24.6, 23.4, 23.1, 21.0, 19.7, 13.6. IR spectrum (CHCl₃): 3402 (NH); 1734, 1698, 1663 (C = O). MS: ESI m/z 466.3 (100 %, M+Na). HR-MS (ESI) m/z: for C₂₇H₄₁NO₄Na [M+Na] calcd, 466.29278; found, 466.29283.

### Example 3: (3R,5R,8R,9S,10S,13S,14S,17S)-17-Cyano-10,13-dimethylhexadecahydro-1H-cyclopenta[a]phenanthren-3-yl 5-oxopyrrolidine-2-carboxylate (3)

Compound 3 was prepared according to the General Procedure B (DCM). Starting from 3α-hydroxy-5β-androstan-17β-carbonitrile (250 mg, 0.83 mmol), using L-pyroglutamic acid (139 mg, 1.07 mmol), compound 3 (180 mg, 53%) was obtained by column chromatography on silica gel (20% acetone/chloroform): mp 188-189 °C (chloroform/diethyl ether), [α]_{D}²⁰ +75.5 (c 0.2, CHCl₃). ¹H NMR (400 MHz, CDCl₃): δ 0.91 (3H, s, H-18), 0.96 (3H, s, H-19), 4.25 (1H, ddd, *J* = 8.8, 5.1, 0.7 Hz, H-C2'), 4.80 (1H tt, *J* = 11.3, 4.8 Hz, H-3), 5.92 (1H, s, N-H). ¹³C NMR (101 MHz, CDCl₃): δ 177.7, 171.5, 121.4, 75.8, 55.6, 54.5, 44.7, 41.8, 40.5, 40.5, 37.4, 36.3, 35.1, 34.8, 32.2, 29.3, 26.8, 26.8, 26.6, 26.4, 25.0, 24.7, 23.3, 20.6, 14.8. IR spectrum (CHCl₃): 2237 (CN), 1735, 1705 (C = O), 1229 (C-O). MS: ESI m/z 435.3 (100%, M+Na). HR-MS (ESI) m/z: for C₂₅H₃₆N₂O₃Na[M+Na] calcd, 435.26181; found, 345.26135. For C₂₅H₃₆N₂O₃ (412.6) calcd: 72.78%, C; 8.80%, H; 6.79%, N. Found: 72.39%, C; 8.64%, H; 6.33%, N.

### Example 4: (3R,5R,8S,9S,10S,11R,13S,14S,17S)-17-Acetyl-11-hydroxy-10,13-dimethylhexadecahydro-1H-cyclopenta[a]phenanthren-3-yl 5-oxopyrrolidine-2-carboxylate (4)

Compound **4** was prepared according to the General Procedure A (DMF). Starting from 20-oxo-5β-pregnan-3α,11α-diol (334 mg, 1.0 mmol), using L-pyroglutamic acid (193 mg, 1.5 mmol), compound **4** (213 mg, 48%) was obtained by column chromatography on silica gel (30% acetone in CHCl₃): mp 183-185 °C (chloroform/diethyl ether), [α]_{D}²⁰ +86.4 (c 0.2, CHCl₃). ¹H NMR (400 MHz, CDCl₃): δ 0.62 (3H, s, H-18), 1.06 (3H, s, H-19), 2.13 (1H, s, H-21), 3.91 (1H, s, H-11), 4.20 (1H, ddd, *J* = 8.7, 5.2, 0.7 Hz, H-C2'), 4.85 (1H, tt, *J* = 10.9, 5.1 Hz, H-3), 5.83 (1H, s, N-H). ¹³C NMR (101 MHz, CDCl₃): δ 209.1, 177.7, 171.5, 76.3, 69.1, 63.5, 55.7, 55.6, 50.8, 47.3, 44.3, 43.5, 38.0, 35.9, 34.8, 32.7, 31.6, 29.3, 27.6, 27.4, 26.3, 25.0, 24.5, 23.7, 23.1, 14.6. IR spectrum (CHCl₃): 1734, 1702 (C = O). MS: ESI m/z 468.3 (100%, M+Na). HR-MS (ESI) m/z: for C₂₆H₃₉NO₅Na [M+Na] calcd, 468.27219; found, 468.27204. For C₂₆H₃₉NO₅ (445.6) calcd: 70.08%, C; 8.82%, H; 3.14%, N. Found: 69.91%, C; 8.68%, H; 2.87%, N.

### Example 5: (3R,5R,8S,9S,10S,13S,14S)-10,13-Dimethylhexadecahydro-1H-cyclopenta[a]phenanthren-3-yl 5-oxopyrrolidine-2-carboxylate (5)

Compound 5 was prepared according to the General Procedure A (DFM). Starting from 3α-hydroxy-5β-androstane (300 mg, 0.94 mmol), using L-pyroglutamic acid (190 mg, 1.5 mmol), compound 5 (210 mg, 58%) was obtained by column chromatography on silica gel (5% acetone/chloroform): mp 136-137 °C (chloroform/diethyl ether), [α]_{D}²⁰ +29.5 (c 0.2, CHCl₃). ¹H NMR (400 MHz, CDCl₃): δ 0.69 (3H, s, H-18), 0.94 (3H, s, H-19), 4.16-4.24 (1H, m, H-C2'), 4.79 (1H tt, *J* = 11.3, 4.8 Hz, H-3), 5.89 (1H, s, N-H). ¹³C NMR (101 MHz, CDCl₃): δ 177.5, 171.4, 76.0, 55.5, 54.5, 41.9, 40.9, 40.7, 40.5, 39.0, 36.2, 35.0, 34.7, 32.1, 29.2, 27.0, 26.7, 26.5, 25.5, 24.9, 23.3, 20.8, 20.6, 17.5. IR spectrum (CHCl₃): 1734, 1704 (C = O). MS: ESI m/z 386.3 (100%, M-H). HR-MS (ESI) m/z: for C₂₄H₃₆NO₃ [M-H] calcd, 386.27007; found, 386.26962. For C₂₄H₃₇NO₃ (387.3) calcd: 74.38%, C; 9.62%, H; 3.61%N. Found: 74.20%, C; 9.61%, H; 3.26%, N.

### Example 6: (3R,5R,8R,9S,10S,13S,14S)-10,13-Dimethyl-17-oxohexadecahydro-1H-cyclopenta[a]phenanthren-3-yl 5-oxopyrrolidine-2-carboxylate (6)

Compound **6** was prepared according to the General Procedure A (DMF). Starting from 17-oxo-5β-androstan-3α-ol (290 mg, 1.0 mmol), using *L*-pyroglutamic acid (194 mg, 1.5 mmol), compound **6** (197 mg, 49%) was obtained by column chromatography on silica gel (5% acetone/chloroform): mp 114-116 °C (acetone/n-heptane), [α]_{D}²⁰ +98.5 (c 0.2, CHCl₃). ¹H NMR (400 MHz, CDCl₃): δ 0.86 (3H, s, H-18), 0.97 (3H, s, H-19), 4.20 (1H, m, H-C2'), 4.80 (1H, m, H-3), 5.91 (1H, s, N-H). ¹³C NMR (101 MHz, CHCl₃): δ 221.3, 177.7, 171.5, 75.8, 55.6, 51.6, 47.9, 41.9, 40.9, 36.1, 35.5, 35.0, 34.9, 32.2, 31.8, 29.3, 26.8, 26.6, 25.4, 25.0, 23.3, 21.9, 20.3, 13.9. IR spectrum (CHCl₃): 3439 (NH); 1703, 1706 (C = O); 1060 (C-O). MS: ESI m/z 424.2 (100%, M+Na). HR-MS (ESI) m/z: for C₂₄H₃₅NO₄Na [M+Na] calcd, 424.24583; found, 424.24548. For C₂₄H₃₅NO₄ (401.2) calcd: 71.79%, C; 8.79%, H; 3.49%, N. Found: 71.40%, C; 8.93%, H, 3.32%, N.

### Example 7: (3R,5R,8S,9S,10S,13S,14S)-10,13-Dimethylhexadecahydro-1H-cyclopenta[a]phenanthren-3-yl 6-oxopiperidine-2-carboxylate (7)

Compound **7** was prepared according to the General Procedure A (DMF). Starting from 5β-androstan-3α-ol (276 mg, 1.0 mmol), using 6-oxo-L-pipecolic acid (213 mg, 1.5 mmol), compound **7** (189 mg, 47%) was obtained by column chromatography on silica gel (5% acetone/chloroform): mp 109-111 °C (diethyl ether), [α]_{D}²⁰ +18.3 (c 0.3, CHCl₃). ¹H NMR (400 MHz, CDCl₃): δ 0.68 (3H, s, H-18), 0.94 (3H, s, H-19), 4.04 (1H, m, H-C2'), 4.81 (1H, m, H-3), 6.13 (1H, s, N-H). ¹³C NMR (101 MHz, CHCl₃): δ 171.4, 170.6, 76.3, 55.1, 54.7, 42.0, 41.1, 40.9, 40.6, 39.1, 36.3, 35.2, 34.9, 32.3, 31.2, 27.1, 26.8, 26.7, 25.7, 25.6, 25.6, 23.4, 21.0, 20.7, 19.7, 17.6. IR spectrum (CHCl₃): 3402 (NH); 1734, 1665 (C = O); 1062 (C-O). MS: ESI m/z 424.3 (100%, M+Na). HR-MS (ESI) m/z: for C₂₅H₃₉NO₃Na [M+Na] calcd, 424.28222; found, 424.28258. For C₂₅H₃₉NO₃ (401.3) calcd: 74.77%, C; 9.79%, H; 3.49%, N. Found: 74.97%, C; 9.92%, H; 3.43%, N.

### Example 8: (3R,5R,8R,9S,10S,13S,14S,17S)-17-Cyano-10,13-dimethylhexadecahydro-1H-cyclopenta[a]phenanthren-3-yl 6-oxopiperidine-2-carboxylate (8)

Compound **8** was prepared according to the General Procedure A (DMF). Starting from 3α-hydroxy-5β-androstan-17β-carbonitrile (112 mg, 0.37 mmol), using 6-oxo-L-pipecolic acid (80 mg, 0.56 mmol), compound **8** (76 mg, 48%) was obtained by column chromatography on silica gel (3% acetone/chloroform): mp 69-71 °C (ethyl acetate/n-heptane), [α]_{D}²⁰ +65.4 (c 0.3, CHCl₃). ¹H NMR (400 MHz, CDCl₃): δ 0.91 (3H, s, H-19), 0.95 (3H, s, H-18), 2.29 (1H, t, *J* = 8.8 Hz, H-17), 4.04 (1H, m, H-C2'), 4.81 (1H, m, H-3), 6.12 (1H, s, N-H). ¹³C NMR (101 MHz, CHCl₃): δ 171.4, 170.5, 121.4, 75.9, 55.1, 54.5, 44.7, 41.8, 40.5, 40.5, 37.4, 36.3, 35.0, 34.8, 32.2, 31.2, 26.8, 26.8, 26.7, 26.4, 25.6, 24.7, 23.3, 20.7, 19.7, 14.5. IR spectrum (CHCl₃): 3403 (NH); 2237 (CN); 1734, 1664, 1418 (C = O); 1062 (C-O). MS: ESI m/z 449.3 (100%, M+Na). HR-MS (ESI) m/z: for C₂₆H₃₈N₂O₃Na [M+Na] calcd, 449.27746; found, 449.27789. For C₂₆H₃₈N₂O₃ (426.6) calcd: 73.20%, C; 8.98%, H; 6.57%, N. Found: 73.22%, C; 9.29%, H; 6.25%, N.

### Example 9: (3R,5R,8R,9S,10S,13S,14S,17S)-17-Acetyl-10,13-dimethylhexadecahydro-1H-cyclopenta[a]phenanthren-3-yl acetylglycinate (9)

Compound **9** was prepared according to the General Procedure A (DMF). Starting from 20-oxo-5β-pregnan-3α-ol (318 mg, 1.0 mmol), using *N*-acetylglycine (176 mg, 1.5 mmol), compound **9** (338 mg, 81%) was obtained by column chromatography on silica gel (3% acetone/chloroform): mp 111-113 °C (ethyl acetate/n-heptane), [α]_{D}²⁰ +92.0 (c 0.3, CHCl₃). ¹H NMR (400 MHz, CDCl₃): δ 0.60 (3H, s, H-18), 0.93 (3H, s, H-19), 2.04 (3H, s, H-C5'), 2.11 (3H, s, H-21), 2.53 (1H, t, *J* = 8.8 Hz, H-17), 4.00 (2H, d, *J =* 5.0 Hz, H-C5'), 4.80 (1H, m, H-3), 6.00 (1H, m, N-H). ¹³C NMR (101 MHz, CHCl₃): δ 209.7, 170.2, 169.7, 75.9, 63.9, 56.8, 44.4, 41.9, 41.8, 40.6, 39.3, 35.9, 35.1, 34.8, 32.3, 31.7, 27.0, 26.7, 26.4, 24.6, 23.4, 23.2, 23.0, 21.0, 13.6. IR spectrum (CHCl₃): 3463, 1516 (NH); 1734, 1697, 1676 (C = O); 1192, 1063 (C-O). MS: ESI m/z 440.3 (100%, M+Na). HR-MS (ESI) m/z: for C₂₅H₃₉NO₄Na [M+Na] calcd, 440.27713; found, 440.27753. For C₂₅H₃₉NO₄ (417.6) calcd: 71.91%, C; 9.41%, H; 3.35%, N. Found: 72.03%, C; 9.36%, H; 3.39%, N.

### Example 10: (3R,5R,8R,9S,10S,13S,14S,17S)-17-Acetyl-10,13-dimethylhexadecahydro-1H-cyclopenta[a]phenanthren-3-yl acetylleucinate (10)

Compound **10** was prepared according to the General Procedure A (DMF). Starting from 20-oxo-5β-pregnan-3α-ol (318 mg, 1.0 mmol), using *N*-acetyl-*L*-leucine (260 mg, 1.5 mmol), compound **10** (155 mg, 33%) was obtained by column chromatography on silica gel (15% ethyl acetate/petroleum ether) as a mixture of diasteromers, followed by HPLC purification (20% acetone/hexane) affording C3'-R/S (unidentified) diastereomer as an oily product: [α]_{D}²⁰ +93.3 (c 0.3, CHCl₃). ¹H NMR (400 MHz, CDCl₃): δ 0.60 (3H, s, H-18), 0.93 (3H, s, H-19), 0.95 (6H, dd, *J* = 6.3, 4.5 Hz, H-C5'and C6'), 2.02 (3H, s, H-acetyl-L-leucinate), 2.11 (3H, s, H-21), 2.55 (1H, t, *J* = 8.8 Hz, H-17), 4.58 (1H, m, H-C2'), 4.76 (1H, m, H-3), 5.83 (1H, m, NH). ¹³C NMR (101 MHz, CHCl₃): δ 209.8, 173.0, 169.9, 75.6, 64.0, 56.8, 51.1, 44.5, 42.0, 40.6, 39.3, 35.9, 35.1, 34.8, 32.2, 31.7, 27.0, 26.7, 26.5, 25.0, 24.6, 23.4, 23.4, 23.0, 23.0, 22.3, 21.0. IR spectrum (CHCl₃): 3436 (NH); 2959, 2872 (CH₃); 1727, 1697 (C = O); 1193, 1021 (C-O). MS: ESI m/z 496.3 (100%, M+Na). HR-MS (ESI) m/z: for C₂₉H₄₈NO₄ [M+H] calcd, 474.35779; found, 474.35753. For C₂₉H₄₇NO₄ (473.7) calcd: 73.53%, C; 10.00%, H; 2.96%, N. Found: 73.37%, C; 10.35%, H; 2.69%, N.

### Example 11: (3R,5R,8S,9S,10S,13R,14S,17S)-10,13,17-Trimethylhexadecahydro-1H-cyclopenta[a]phenanthren-3-yl 5-oxopyrrolidine-2-carboxylate (11)

Compound 11 was prepared according to the General Procedure A (DMF). Starting from 17β-methyl-5β-androstan-3α-ol (102 mg, 0.35 mmol), using *L*-pyroglutamic acid (112 mg, 0.525 mmol), compound 11 (124 mg, 60%) was obtained as a white amorphous solid by column chromatography on silica gel (acetone/chloroform, 1:50 to 1:6): mp 157-158 °C (acetone/n-heptane), [α]_{D}²⁰+31.0 (c 0.3, CHCl₃). ¹H NMR (400 MHz, CDCl₃): δ 0.52 (3H, s, H-18), 0.82 (3H, d, *J =* 6.8 Hz, 17-Me), 0.94 (3H, s, H-19), 4.19 (dd, *J* = 8.6, 5.2 Hz, H-C2'), 4.78 (1H, tt, *J* = 11.4, 4.8 Hz, H-3), 6.19 (1H, s, N-H). ¹³C NMR (101 MHz, CDCl₃): δ 177.9, 171.6, 76.1, 55.9, 55.7, 45.3, 42.3, 42.2, 40.9, 37.8, 36.2, 35.2, 34.8, 32.3, 30.4, 29.4, 27.2, 26.7, 26.6, 25.0, 24.8, 23.5, 20.7, 13.9, 12.2. IR spectrum (CHCl₃): 3206, 3118 (NH), 2951, 2867 (CH₂), 1736 (C = O), 1716 (C = O), 1448 (CH₂), 1203, 1022 (C-O). MS: ESI m/z 424.3 (100%, M+Na), 402.3 (24%, M+H). HR-MS (ESI) m/z: for C₂₅H₃₉NO₃Na [M+Na] calcd, 424.2823, found, 424.2822; for C₂₅H₄₀NO₃ [M+H] calcd, 402.3003, found, 402.3003. For C₂₅H₃₉NO₃ (401.6) calcd: 74.77%, C; 9.79%, H; 3.49%, N. Found: 74.77%, C; 9.94%, H; 2.94%, N.

### Example 12: (3R,5R,8R,9S,10S,13S,14S,Z)-17-Ethylidene-10,13-dimethylhexadecahydro-1H-cyclopenta[a]phenanthren-3-yl 5-oxopyrrolidine-2-carboxylate (12)

Compound **12** was prepared according to the General Procedure A (DMF). Starting from 5β-pregnan-17-ethylidene-3α-ol (302 mg, 1.0 mmol), using *L*-pyroglutamic acid (194 mg, 1.5 mmol). The crude product was pre-purified by column chromatography on silica gel (8% acetone/chloroform). Compound **12** (190 mg, 46%) was obtained by HPLC separation in the following setting. High Pressure Pump (model 361, Gilson), Inject Valve Rheodyne, preparative ELSD Detector (Gilson) connected with PC (software Trilution LC, Gilson). Flow rate 17 mL/min, acetone/hexane 20/80, elution time 48 min. Column Luna 5 µm Si(2); Axia Packed 250x21.2 mm. Loading: 1 mL of dichloromethane: mp 155-157 °C (acetone/hexane), [α]_{D}²⁰ +57.1 (c 0.3, CHCl₃). ¹H NMR (400 MHz, CDCl₃): δ 0.86 (3H, s, H-18), 0.95 (3H, s, H-19), 4.20 (1H, ddd, *J* = 8.7, 5.4, 0.7 Hz, H-C2'), 4.79 (1H, tt, *J* = 11.4, 4.8 Hz, H-3), 5.11 (1H, qt, *J* = 7.1, 2.0 Hz, H-20), 5.86 (1H, s, N-H). ¹³C NMR (101 MHz, CDCl₃): δ 177.6, 171.6, 150.4, 113.4, 76.1, 56.4, 55.6, 44.6, 42.0, 40.7, 37.5, 35.5, 35.0, 34.8, 32.3, 31.6, 29.3, 27.1, 26.7, 26.3, 25.0, 24.5, 23.4, 21.2, 17.0, 13.3. IR spectrum (CHCl₃): 3438, 1706 (oxopyrrolidine); 1734 (C = O); 1244, 1022 (C-O); 1678, 828 (C=C). MS: ESI m/z 827.6 (100%, 2M), 414.3 (65%, M+H). HR-MS (ESI) m/z: for C₂₆H₄₀NO₃ [M+H] calcd, 414.30027; found, 414.29987. For C₂₆H₃₉NO₃ (413.6) calcd: 75.50%, C; 9.50%, H; 3.39%, N. Found: 75.24%, C; 9.51%, H; 3.02%, N.

### Example 13: 2-(((3R,5R,8S,9S,10S,13S,14S)-10,13-Dimethylhexadecahydro-1H-cyclopenta[a]phenanthren-3-yl)oxy)ethyl 5-oxopyrrolidine-2-carboxylate (13)

Compound **13** was prepared according to the General Procedure A (DMF). Starting from 3-(5β-androstan-3α-yl)oxy)ethan-1-ol (320 mg, 1.0 mmol), using L-pyroglutamic acid (194 mg, 1.5 mmol). The crude product was pre-purified by column chromatography on silica gel (8% acetone/chloroform). Compound **13** (180 mg, 42%) was obtained by HPLC separation in the following setting. High Pressure Pump (model 361, Gilson), Inject Valve Rheodyne, preparative ELSD Detector (Gilson) connected with PC (software Trilution LC, Gilson). Flow rate 17 mL/min, acetone/hexane 20/80, elution time 45 min. Column Luna 5 µm Si(2); Axia Packed 250x21.2 mm. Loading: 1 mL of dichloromethane: mp 110-111 °C (acetone/hexane), [α]_{D}²⁰ +11.4 (c 0.3, CHCl₃). ¹H NMR (400 MHz, CDCl₃): δ 0.67 (3H, s, H-18), 0.92 (3H, s, H-19), 3.28 (1H, tt, *J* = 11.1, 4.6 Hz, H-3), 3.48 (1H, q, *J* = 7.0 Hz, H-C2'), 3.64-3.74 (2H, m, OCH2CH₂O-ster), 4.18-4.42 (2H, m, OCH₂CH₂O-ster), 5.92 (1H, s, N-H). ¹³C NMR (101 MHz, CDCl₃): δ 177.6, 172.1, 79.9, 65.7, 65.3, 55.4, 54.7, 42.3, 41.1, 40.8, 40.6, 39.2, 36.4, 35.6, 35.2, 33.3, 29.2, 27.4, 27.3, 26.9, 25.7, 25.0, 23.6, 21.0, 20.7, 17.6. IR spectrum (CHCl₃): 3437, 1705 (oxopyrrolidine); 1744 (C = O); 1240, 1033 (C-O). MS: ESI m/z 454.3 (87%, M+Na), 432.3 (58%, M+H). HR-MS (ESI) m/z: for C₂₆H₄₂NO₄ [M+H] calcd, 432.31084; found, 432.31049. For C₂₆H₄₁NO₄ (431.6) calcd: 72.35%, C; 9.58%, H; 3.25%, N. Found: 71.99%, C; 9.93%, H; 3.13%, N.

### Example 14: 2-(((3R,5R,8S,9S,10S,13S,14S)-10,13-Dimethylhexadecahydro-1H-cyclopenta[a]phenanthren-3-yl)oxy)ethyl 6-oxopiperidine-2-carboxylate (14)

Compound **14** was prepared according to the General Procedure A (DMF). Starting from 3-(5β-androstan-3α-yl)oxy)ethan-1-ol (320 mg, 1.0 mmol), using 6-oxo-*L*-pipecolic acid (214 mg, 1.5 mmol). The crude product was pre-purified by column chromatography on silica gel (5% acetone/chloroform). Compound **14** (170 mg, 38%) was obtained by HPLC separation in the following setting. High Pressure Pump (model 361, Gilson), Inject Valve Rheodyne, preparative ELSD Detector (Gilson) connected with PC (software Trilution LC, Gilson). Flow rate 17 mL/min, acetone/hexane 20/80, elution time 42 min. Column Luna 5 µm Si(2); Axia Packed 250x21.2 mm. Loading: 1 mL of dichlormethane: mp 117-119 °C (acetone/hexane), [α]_{D}²⁰ +8.1 (c 0.3, CHCl₃). ¹H NMR (400 MHz, CDCl₃): δ 0.68 (3H, s, H-18), 0.92 (3H, s, H-19), 3.27 (1H, tt, *J =* 11.1, 4.6 Hz, H-3), 3.69 (2H, ddd, *J =* 5.5, 4.2, 1.3 Hz, OCH₂CH₂O-ster), 4.12 (1H, ddd, *J* = 8.3, 5,1, 1,6 Hz, H-C2'), 4.25-4.36 (2H, m, OCH₂CH₂O-ster), 6.11 (1H, s, N-H). ¹³C NMR (101 MHz, CDCl₃) δ 171.0, 170.9, 79.6, 65.3, 65.1, 54.6, 54.4, 42.0, 40.8, 40.5, 40.3, 38.9, 36.0, 35.2, 34.8, 33.0, 30.9, 27.1, 26.9, 26.6, 25.4, 25.3, 23.2, 20.7, 20.4, 19.4, 17.3. IR spectrum (CHCl₃): 3400, 1666 (oxopiperidine); 1743 (C = O); 1294, 1244 (C-O). MS: ESI m/z 468.3 (100%, M+Na), 446.3 (88%, M+H). HR-MS (ESI) m/z: for C₂₇H₄₄NO₄ [M+H] calcd, 446.32649; found, 446.32614. For C₂₇H₄₃NO₄ (445.6) calcd: 72.77%, C; 9.73%, H; 3.14%, N. Found: 72.63%, C; 9.65%, H; 3.03%, N.

### Example 15: 1-((3R,5R,8R,9S,10S,13S,14S,17S)-3-(2-Hydroxyethyl)-10,13-dimethylhexadecahydro-1H-cyclopenta[a]phenanthren-17-yl)ethan-1-one (15)

A solution of 20-oxo-5β-pregnan-3α-acetic acid (5.9 g, 16.0 mmol) in THF (20 mL) was added dropwise to the refluxing solution of LiAlH₄ (1.4 g, 36.8 mmol) in THF (580 mL). After 2 hrs of reflux, the reaction mixture was cooled to 0 °C, quenched with saturated aqueous solution of Na₂SO₄ and solids were filtered off. The filtrate was concentrated *in vacuo* and the residue was diluted with ethyl acetate. The combined extracts were washed with aqueous solution of hydrochloric acid (10%; 3 x 40 mL), water (3 x 40 mL), and saturated solution of NaHCO₃ (3 x 40 mL). Solvents were dried over Na₂SO₄ and evaporated. Purification by column chromatography on silica gel (4% ethyl acetate/petroleum ether) gave hydroxyl derivative (3.1 g, 54%) as a mixture of 20R and 20S-isomers that was used for the next reaction step. Aqueous solution of NaOCl (5.5%, 30 mL) was added into a solution of hydroxy derivative (3 g, 8.6 mmol) in acetic acid (75 mL). After stirring at room temperature for 1 hour, isopropanol was added (45 mL) and the reaction mixture was stirred for additional 30 min. Then, it was diluted with water and extracted with chloroform (3 x 30 mL). Combined extracts were washed with brine, dried over anhydrous Na₂SO₄ and the solvents evaporated. Column chromatography on silica gel (3-5% acetone/hexane) was followed by HPLC separation in the following setting. High Pressure Pump (model 361, Gilson), Inject Valve Rheodyne, preparative ELSD Detector (Gilson) connected with PC (software Trilution LC, Gilson). Flow rate 10 mL/min, acetone/hexane 20/80, elution time 36 min. Column Luna 5 µm Si(2); Axia Packed 250x21.2 mm. Loading: 3 mL of dichloromethane. Compound **15** (850 mg, 25%) was used in the next reaction step: ¹H NMR (400 MHz, CDCl₃): δ 0.59 (3H, s, H-18), 0.92 (3H, s, H-19), 2.11 (3H, s, H-21), 2.53 (1H, t, *J =* 9.0 Hz, H-17), 3.66 (2H, t, *J =* 6.9 Hz, O-CH₂-CH-ster). ¹³C NMR (101 MHz, CDCl₃) δ 209.6, 63.8, 61. 7, 56.7, 44.2, 39.9, 39.2, 37.3, 35.6, 35.3, 34.6, 31.4, 31.1, 30.4, 29.5, 27.0, 26.2, 24.6, 24.3, 24.0, 22.7, 20.8, 13.3.

### Example 16: 2-((3R,5R,8R,9S,10S,13S,14S,17S)-17-Acetyl-10,13-dimethylhexadecahydro-1H-cyclopenta[a]phenanthren-3-yl)ethyl 5-oxopyrrolidine-2-carboxylate (16)

Compound **16** was prepared according to the General Procedure A (DMF). Starting from 20-oxo-5β-pregnan-3α-ethanol (347 mg, 1 mmol), using L-pyroglutamic acid (194 mg, 1,5 mmol). The crude product was pre-purified by column chromatography on silica gel (8% acetone/chloroform). Compound **16** (200 mg, 44%) was obtained by HPLC separation in the following setting. High Pressure Pump (model 361, Gilson), Inject Valve Rheodyne, preparative ELSD Detector (Gilson) connected with PC (software Trilution LC, Gilson). Flow rate 10 mL/min, acetone/hexane 40/80, elution time 42 min. Column Luna 5 µm Si(2); Axia Packed 250x21.2 mm. Loading: 3 mL of dichloromethane: low melting point solid, [α]_{D}²⁰ +66.3 (c 0.3, CHCl₃). ¹H NMR (400 MHz, CDCl₃): δ 0.59 (3H, s, H-18), 0.93 (3H, s, H-19), 2.11 (3H, s, H-21), 4.18 (2H, t, *J* = 6.9 Hz, O-CH₂-CH-ster), 4.26-4.21 (1H, m, H-C2'), 5.90 (1H, s, N-H). ¹³C NMR (101 MHz, CDCl₃): δ 209.8, 177.6, 172.1, 65.1, 64.1, 57.0, 55.5, 44.5, 40.2, 39.5, 37.6, 35.9, 35.6, 31.7, 31.3, 30.5, 30.2, 29.3, 27.2, 26.5, 25.0, 25.0, 24.7, 24.6, 24.3, 23.0, 21.1, 13.6. IR spectrum (CHCl₃): 3438, 1739, 1702 (oxopyrrolidine); 2924, 2864 (CH₂). MS: ESI m/z 480.3 (100%, M+Na), 458.3 (12%, M+H). HR-MS (ESI) m/z: for C₂₈H₄₄NO₄ [M+H] calcd, 458.32649; found, 458.32611. For C₂₈H₄₃NO₄ (457.7) calcd: 73.49%, C; 9.47%, H; 3.06%, N. Found: 73.08%, C; 9.43%, H; 2.79%, N.

### Example 17: 2-((3R,5R,8R,9S,10S,13S,14S,17S)-17-Acetyl-10,13-dimethylhexadecahydro-1H-cyclopenta[a]phenanthren-3-yl)ethyl 6-oxopiperidine-2-carboxylate (17)

Compound **KK-17** was prepared according to the General Procedure A (DMF). Starting from 20-oxo-5β-pregnan-3α-ethanol (347 mg, 1.0 mmol), using 6-oxo-L-pipecolic acid (214 mg, 1.5 mmol). The crude product was pre-purified by column chromatography on silica gel (5% acetone/chloroform). Compound **KK-17** (175 mg, 37%) was obtained by HPLC purification in the following setting. High Pressure Pump (model 361, Gilson), Inject Valve Rheodyne, preparative ELSD Detector (Gilson) connected with PC (software Trilution LC, Gilson). Flow rate 12 mL/min, acetone/hexane 40/60, elution time 37 min. Column Luna 5 µm Si(2); Axia Packed 250x21.2 mm. Loading: 1 mL of dichlormethane: mp oil, [α]_{D}²⁰ +61.5 (c 0.2, CHCl₃). ¹H NMR (400 MHz, CDCl₃): δ 0.59 (3H, s, H-18), 0.93 (3H, s, H-19), 2.11 (3H, s, H-21), 4.10-4.03 (2H, m, OCH₂CH₂O-ster), 4.30-4.11 (1H, m, H-C2'), 6.11 (1H, s, N-H). ¹³C NMR (101 MHz, CDCl₃) δ 209.8, 171.3, 171.2, 164.4, 65.2, 64.1, 57.0, 55.0, 44.5, 40.2, 39.5, 37.5, 35.9, 35.6, 31.7, 31.3, 31.2, 30.5, 30.2, 27.9, 27.2, 26.5, 25.6, 24.6, 24.3, 23.0, 21.1, 19.7, 13.6. IR spectrum (CHCl₃): 3102 (oxopiperidine); 1739, 1697 (C = O); 1265, 1011 (C-O). MS: ESI m/z 494.3 (100%, M+Na). HR-MS (ESI) m/z: for C₂₉H₄₅NO₄Na [M+Na] calcd, 494.32408; found, 494.32371.

### Experiments in vivo

Adults wild-type (strain AB) zebrafish *(Danio rerio)* were maintained at 28-29 °C on a light cycle of 14h light:10h dark (lights on at 7 am; lights off at 9 pm) at ZeClinics animal facility (Barcelona). All procedures involving animals and their care were conducted accordingly to CEA-OH/9421/2 authorization from Government of Catalonia. Embryos obtained by pairwise mating were raised in E3 media at the controlled temperature of 28.5 °C until 7 days post-fertilization (dpf) when the experiments were performed.

Anticonvulsant effect of compounds was evaluated in two models of epileptic seizures in immature rats (PTZ model and 6 Hz model). PTZ convulsions were tested in 12- and 25-day-old rats, 6 Hz-induced seizures in 15- and 25-day-old rats. Young adult rats (P60) were also used in either test. Day of birth was taken as P0. Experiments evaluating precognitive effect were performed in adult male rats of the Wistar and Long Evans strain (3 months old, b.w. 300-400 g). All animals were obtained from a breeding facility of Institute of Physiology CAS (certificate No. 1396/2014-MZE-17214). Animals were held in standard conditions (21±1°C, humidity 50-60%, light regime 12/12) with unrestricted access to food and water. All manipulations with animals were performed according to the https://nc3re.org.uk/arrive-guidelines and to Czech Animal Protection Law 246/1992 Sb. And international directives (EU Directive 2010/63/EU for animals' experiments).

Compound from Example 1 was selected to test anticonvulsant and neuroprotective effects in relation to an ethical demand for reduction of number of laboratory animals in experiments.

### Anticonvulsant effect of steroid compounds in the model of pentetrazol-induced seizures in zebrafish (Danio rerio)

The anticonvulsant activity of the claimed compounds on the nervous system of zebrafish *(Danio rerio).* This test is the modern alternative to tests in adult animals as *Danio rerio* is very versatile organism that has a number of physiological similarities to mammals, including humans (Expert Opin. Drug Metab. Toxicol. 2009, 5, 393). Steroidal compounds were tested in three concentrations (1, 3 and 5 µmol.L⁻¹) on 7 days post-fertilization (dpf) wild-type larvae. As a negative control, sibling larvae were incubated with DMSO 1%, which allows us to detect behavioral alterations of PTZ treated larvae. Seizures were induced by treatment of pentetrazol (PTZ) at the dose 5 mmol.L⁻¹. Commercially available anticonvulsant drug Topiramate was used as comparator. Results were analyzed with GraphPad Prism software and statistical significance is assessed by the One-way ANOVA statistical analysis followed by Tukey's multiple comparisons test. The zebrafish larvae locomotion and response to visual stimuli are traced and analyzed by the EthoVision XT 12 software and the DanioVision device from Noldus Information Technologies, Wageningen, The Netherlands.

This closed system consists of a camera placed above a chamber with circulating water and a temperature sensor that is set at 28 °C. Individualized larvae in a 48-wells plate are placed in the chamber, which can provide different stimuli (light/dark environment, tapping, sound) controlled by the software. Prior to each experiment, larvae were left for 10 minutes in dark for acclimation, then predetermined series of alternating dark and light environment are presented to the larvae. The final experimental protocol is divided in three main part: first a 15 minutes step with light on, then a series of 5 short flashes of light to induce epileptic seizures and finally a 25 minutes dark/light alternating environments phase. Different sets of information can be extrapolated from the different phases: while the first step is useful to detect changes in the total larvae locomotion, the second part allows us to analyze the larval response to a seizure-inducing visual stimulus and measure the seizure characteristics: the maximum velocity and the number of angles turns (specific of seizure erratic movement). The final phase is useful to detect anomalies in larval movement and deviations from the stereotyped behavior (natural locomotor behavior of zebrafish is active in dark and immobile in light). The anticonvulsant effect of compounds was studied at doses of 1, 3 and 5 mg/kg for the compound shown in Example 1, Example 2 and Example 3. The results (Fig. 1 and Fig. 2) indicate that all substances significantly reduced the locomotor activity epileptiform induced by PTZ in the three evaluated parameters - spontaneous locomotor activity, maximum velocity and the angle turns. All substances also decreased epileptiform locomotor activity in the dark phase/light test without causing sedation.

### Anticonvulsant effects of steroid derivatives in rats

Two age groups of immature male Wistar rats were used in either experiment. In PTZ model P12 corresponding by a maturation of the brain to early postnatal human babies, *i.e.* to the period where children epileptic encephalopathy mostly resistant to present pharmacotherapy start to appear. The other group - P25 animals corresponds with school-age children. Generalized tonic-clonic seizures were elicited by a subcutaneous injection of PTZ in a dose of 100 mg/kg. The 6 Hz model was tested in P15 (at this age eyes are open and it is not necessary to surgically distract lids). Biphasic pulses with 1-s duration were applied transcorneally for 3 s by a constant current stimulator of Ugo Basile company. PTZ induced generalized seizures represent a model of human generalized tonic-clonic seizures, seizures elicited by 6 Hz stimulation are generally taken as a model of temporal (psychomotor, complex partial) seizures. Differences in latencies and seizure severity were evaluated by ANOVA with a subsequent pairwise comparison by Holm-Sidak test. Incidence of seizures were evaluated by Fischer exact test. Critical p value was set at p=0.05.

### Effects of steroid derivatives in PTZ-induced seizures

Anticonvulsant effect was studied in 12- and 25-day-old male Wistar rats. Steroid derivatives were administered at doses of 1, 5 and 10 mg/kg *i.p.* 20 min before the administration of PTZ (100 mg/kg *s.c.*).

Anticonvulsant effect of the compound from Example 1 is presented at **Fig. 3****.** Twelve-day-old rats: The 1-mg/kg dose suppressed the tonic phase of GTCS, the 5-mg/kg dose significantly decrease the incidence of seizures and the 10-mg/kg dose completely suppressed the seizures. The 1- and 5-mg/kg doses significantly prolonged latencies to GTCS. Twenty-five-day-old rats: The 5- and 10-mg/kg doses decrease the incidence of GTCS without a selective effect against the tonic phase. Latencies of seizures were not changed after the 1-mg/kg dose, the two higher doses elicited seizures in one animal only. Seizure severity copied the presence of GTCS. Anticonvulsant effect of the compound from Example 2 is presented in **Fig. 4****.** Compound from Example 2 in doses of 5 and 10 mg/kg suppressed significantly incidence of GTCS and seizure severity in both age groups. Anticonvulsant effect of compound from the Example 3 is shown in Fig. 5. This substance in the 5- and 10-mg/kg doses suppressed incidence of GTCS in 25-day-old rats. The 12-day-old group was significantly affected only by the 5-mg/kg dose. Anticonvulsant effect of the compound from Example 1 in adult rats is demonstrated in Fig. 6. The 10-mg/kg dose significantly decreased the incidence of generalized tonic-clonic seizures (GTCS), whereas minimal clonic seizures remained unaffected. Latency to GTCS was significantly decreased by the 5-mg/kg dose in both animals exhibiting these seizures. GTCS were present in only one rat after the 10-mg/kg dose. Seizure severity was significantly decreased after either 5- and 10-mg/kg dose.

### Efficiency of steroid derivatives in the 6 Hz seizure model in rats

Younger group was formed by 15-day-old animals - the animals at this age have open eyes and it is not necessary to distract eyelids surgically. The other group was 25 days old. Sensitivity of these two age groups is different, therefore we used current intensities of 40, 60, and 80 mA in 15-day-old rats and 20, 40, and 60 mA in the 25-day-old animals. Stimulations were made in 20-min intervals, 10 min before each stimulation a drop of mesocaine into either eye was used as a local anesthetic. Steroid derivatives in a dose of 10 mg/kg *i.p.* were administered 20 min before the first stimulation. Anticonvulsant effect of the compound from Example 1 is presented in Fig.7. Seizures elicited by 60-mA intensity of stimulation current were suppressed, those elicited by 80 mA not. Duration of seizures was significantly shortened at both current intensities; seizure severity was not affected. Older age group exhibited a tendency to a decreased incidence of seizures. Seizures were significantly shorter and exhibited lower intensity. Figure 8 demonstrates anticonvulsant effect of the substance from Example 1 in adult rats. Changes of the threshold intensity did not reach the level of significance - this tendency was seen after the high dose of 10 mg/kg *i.p.* Comparison of data from immature and adult rats clearly demonstrate higher efficiency and broader spectrum of effects in pediatric models.

### Efficacy of Compound from Example 1 in the models of affective disorder (Epilepsy Comorbidity)

Behavioral models of the affective disorders are based on exposure to stress. Two behavioral tests were performed: the Elevated plus maze test (EPM, Nat. Protoc. 2007, 2, 322) and the Novelty suppressed feeding model (NSF, Interdiscip. Toxicol. 2017, 10, 40).

In the EPM test, the Compound from Example 1 was administered at doses of 1, 3 and 10 mg / kg, *i.p.* 30 minutes before maze testing. Time spent in open arms, closed arms, and central platform were recorded during a 10-min test session. Administration of the Compound from Example 1 at a dose of 10 mg/kg resulted into significant reduction of anxiety parameters compare to the control group. The EPM test demonstrated the anxiolytic properties of the Compound from Example 1.

In the NSF test, the Compound from Example 1 was administered *i.p.* 30 min before the test at doses 0.1; 0.3; 1; 3 and 10 mg/kg. The results showed the significant decrease in latency to feed (anxiolytic parameter) at 0.3 mg/kg. In doses 1 and 3 mg / kg, there was a trend to decrease the latency of feed intake.

### Efficacy of the Compound from Example 1 in the models of schizophrenia-like behavior and procognitive effect

Schizophrenia-like behavior was induced by *i.p.* administration of dizocilpine (MK-801) at a dose of 0.1 mg/kg. The effect of the Compound from Example 1 was tested in Passive avoidance test (Psychopharmacology (Berl). 2016, 233, 2077). This test allows to study memory and learning ability. The Compound from Example 1 was applied for 30 min *i.p.* before the task in doses of 0.1; 1 and 3 mg/kg. The results show that administration of the Compound from Example 1 to intact animals does not impair memory one hour after application of the aversive stimulus. The effect on reducing the latency to entry was showed for latency measured 24 h after association at a dose of 0.1 mg/kg. Application of dizocilpine resulted in memory impairment, measured as latency to entrance at 1 and 24 h after application of the aversive stimulus. Administration of the Compound of Example 1 at a dose of 0.1 mg/kg prevented memory damage measured 1 h after the inverse stimulus. A similar trend was observed at other doses and for a longer period of time. Thus, the application of the Compound from Example 1 shows a pro-cognitive effect, particularly in short-term memory.

### The neuroprotective effect of the Compound of Example 1 in the model of excitotoxic CNS injury

The neuroprotective effect of the Compound from Example 1 was tested in the model of bilateral excitotoxic lesion of the dorsal hippocampus. The procedure was performed according to literature (Neuropharmacology 2011, 61, 61). This model simulates the overstimulation of NMDA receptors that occurs in the number of pathophysiological states, leading to calcium flow into the neuron's and, consequently, to apoptosis almost necrosis. Clinically, this phenomenon is manifested by neurodegeneration and CNS damage. Rats were randomly divided into three groups. Control animals were those operated animals that were administered phosphate buffer pH 7.4 into the hippocampus. The second group, called NMDA, represented the animals that had been induced NMDA lesions of the hippocampus. Animals in the third group received Compound from Example 1 at dose 1 mg/kg *i.p. 5* minutes after NMDA lesion. NMDA lesion was induced by infusing excitotoxic NMDA (25 mmol.L⁻¹, volume 1 µl) into the dorsal hippocampus, control animals received sterile PBS (10 mmol.L⁻¹). Compounds from Example 1 (1 mg/kg) or vehicle was administered 5 minutes after the end of the NMDA infusion. Transcardial perfusion was performed 24 h after induction of excitotoxic damage to the hippocampus. Brain tissue was then post-fixed overnight in 4% PFA followed by 10%, 20%, 30% (v/v) sucrose (for cryoprotection) for histological evaluation. Histological damage, *e.g.* the neuroprotective effect of Compound from Example 1 was evaluated by staining damaged neurons with Fluoro Jade B (Merck Millipore, Catalog Number AG310-30MG), and evaluated areas included: hippocampus - DG, hilus, CA3, CA1. The results in Fig. 9 show a significant reduction in the dorsal hippocampus damage following administration of the Compound of Example 1. These results clearly demonstrate the neuroprotective effect of the Compound from Example 1.

### Industrial applicability

The compounds from the submitted invention will be useful for treating central nervous system diseases, particularly epilepsy and seizure conditions in children. In addition, they can also be used to influence associated comorbidities. These include depression, anxiety, schizophrenia-like behavior, neurodevelopmental disorders, affective disorders and stress disorders. The claimed substances can be used separately in therapy, but also as adjuvant treatment to medicines currently approved for therapy.

## Claims

1. Compound of general formula I, 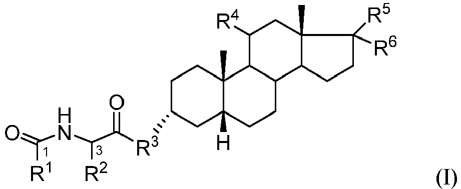 wherein,
- R¹ represents methyl, and then R² is a hydrogen atom or linear or branched C₁-C₄ alkyl, or R¹ and R² form together a group -(CH₂)ₚ-, where p=2 or 3, which forms a five- or six-membered ring together with carbon atoms 1 and 3 and with the nitrogen atom of the general formula I,
- R³ is -O-(CH₂)ₙ-Oₘ-, wherein n = 0, 1 or 2 and m = 0 or 1,
- R⁴ is a hydrogen atom or hydroxyl group,
- R⁵ is a hydrogen atom, and then R⁶ is selected from a group consisting of a hydrogen atom, acetyl group, cyano group, C₁-C₂ cyanoalkyl group, 1,1-difluoroethyl group and linear or branched C₁- C₄ alkyl,
or R⁵ and R⁶ together form a structure selected from a group consisting of C₁- C₂ alkylidene, cyanomethylene group, atom of oxygen, two atoms of fluorine.

2. Compound of general formula I according to Claim 1, selected from a group consisting of:
(3R,5R,8R,9S,10S,13S,14S,17S)-17-Acetyl-10,13-dimethylhexadecahydro-1H-cyclopenta[a]fenantren-3-yl 5-oxopyrrolidine-2-carboxylate (1),
(3R,5R,8R,9S,10S,13S,14S,17S)-17-Acetyl-10,13-dimethylhexadecahydro-1H-cyclopenta[a]phenantren-3-yl 6-oxopiperidine-2-carboxylate (2)
(3R,5R,8R,9S,10S,13S,14S,17S)-17-Cyano-10,13-dimethylhexadecahydro-1H-cyclopenta[a]phenanthren-3-yl 5-oxopyrrolidine-2-carboxylate (3),
(3R,5R,8S,9S,10S,11R,13S,14S,17S)-17-Acetyl-11-hydroxy-10,13-dimethylhexadecahydro-1H-cyclopenta[a]phenanthren-3-yl 5-oxopyrrolidine-2-carboxylate (4),
(3R,5R,8S,9S,10S,13S,14S)-10,13-Dimethylhexadecahydro-1H-cyclopenta[a]phenanthren-3-yl 5-oxopyrrolidine-2-carboxylate (5),
(3R,5R,8R,9S,10S,13S,14S)-10,13-Dimethyl-17-oxohexadecahydro-1H-cyclopenta[a]phenanthren-3-yl 5-oxopyrrolidine-2-carboxylate (6),
(3R,5R,8S,9S,10S,13S,14S)-10,13-Dimethylhexadecahydro-1H-cyclopenta[a]phenanthren-3-yl 6-oxopiperidine-2-carboxylate (7),
(3R,5R,8R,9S,10S,13S,14S,17S)-17-Cyano-10,13-dimethylhexadecahydro-1H-cyclopenta[a]phenanthren-3-yl 6-oxopiperidine-2-carboxylate (8),
(3R,5R,8R,9S,10S,13S,14S,17S)-17-Acetyl-10,13-dimethylhexadecahydro-1H-cyclopenta[a]phenanthren-3-yl acetylglycinate (9),
(3R,5R,8R,9S,10S,13S,14S,17S)-17-Acetyl-10,13-dimethylhexadecahydro-1H-cyclopenta[a]phenanthren-3-yl acetylleucinate (10),
(3R,5R,8S,9S,10S,13R,14S,17S)-10,13,17-Trimethylhexadecahydro-1H-cyclopenta[a]phenanthren-3-yl 5-oxopyrrolidine-2-carboxylate (11),
(3R,5R,8R,9S,10S,13S,14S,Z)-17-Ethylidene-10,13-dimethylhexadecahydro-1H-cyclopenta[a]phenanthren-3-yl 5-oxopyrrolidine-2-carboxylate (12),
2-(((3R,5R,8S,9S,10S,13S,14S)-10,13-Dimethylhexadecahydro-1H-cyclopenta[a]phenanthren-3-yl)oxy)ethyl 5-oxopyrrolidine-2-carboxylate (13),
2-(((3R,5R,8S,9S,10S,13S,14S)-10,13-Dimethylhexadecahydro-1H-cyclopenta[a]phenanthren-3-yl)oxy)ethyl 6-oxopiperidine-2-carboxylate (14),
2-((3R,5R,8R,9S,10S,13S,14S,17S)-17-Acetyl-10,13-dimethylhexadecahydro-1H-cyclopenta[a]phenanthren-3-yl)ethyl 5-oxopyrrolidine-2-carboxylate (16),
2-((3R,5R,8R,9S,10S,13S,14S,17S)-17-Acetyl-10,13-dimethylhexadecahydro-1H-cyclopenta[a]phenanthren-3-yl)ethyl 6-oxopiperidine-2-carboxylate (17).

3. Compound of general formula I according to claim 1 or 2 for use as medicament.

4. Compound of general formula I according to claim 1 or 2 for use in treatment of epilepsy or conditions associated with convulsions, such as seizures associated with hypoxia; seizures associated with traumatic brain damage; seizures associated with intoxication; pathological changes caused by hyperexcitation.

5. Compound of general formula I according to claim 1 of 2 for use in treatment of conditions accompanying epilepsy, such as affective disorders, depression, post-traumatic stress disorder (PTSD) and stress-related diseases, anxiety, schizophrenia and psychotic disorders, related ischemic CNS damage, neurodegenerative changes and disorders, multiple sclerosis.

6. Use of the compound of general formula I according to claim 1 or 2 in the manufacture of a medicament for use in treatment of epilepsy or comorbidities associated with epilepsy or conditions associated with convulsions, such as seizures associated with hypoxia; seizures associated with traumatic brain damage; seizures associated with intoxication; pathological changes caused by hyperexcitation; or in treatment of conditions accompanying epilepsy, such as affective disorders, depression, post-traumatic stress disorder (PTSD) and stress-related diseases, anxiety, schizophrenia and psychotic disorders, related ischemic CNS damage, neurodegenerative changes and disorders, multiple sclerosis.

7. Pharmaceutical composition, **characterized in that** it contains, as an active ingredient, at least one compound of the general formula I according to claim 1 or 2, and at least one pharmaceutically acceptable excipient.

8. Pharmaceutical composition according to Claim 7 for the treatment of epilepsy and comorbidities associated with epilepsy or conditions associated with convulsions, such as seizures associated with hypoxia; seizures associated with traumatic brain damage; seizures associated with intoxication; pathological changes caused by hyperexcitation; or in treatment of conditions accompanying epilepsy, such as affective disorders, depression, post-traumatic stress disorder (PTSD) and stress-related diseases, anxiety, schizophrenia and psychotic disorders, related ischemic CNS damage, neurodegenerative changes and disorders, multiple sclerosis.

9. Compound of claim 1 or 2 for use in a method of treatment of epilepsy and comorbidities associated with it or other conditions associated with convulsions, such as seizures associated with hypoxia; seizures associated with traumatic brain damage; seizures associated with intoxication; pathological changes caused by hyperexcitation; or for the treatment of conditions that may accompany epilepsy, such as affective disorders, depression, post-traumatic stress disorder (PTSD) and stress-related diseases, anxiety, schizophrenia and psychotic disorders, related ischemic CNS damage, neurodegenerative changes and disorders, multiple sclerosis, said method comprising the step of administering at least one compound of general formula I according to claim 1 to a patient in need of such treatment.

10. Compound of general formula I according to claim 1 or 2 for use as active ingredients or auxiliary ingredients in food supplements or cosmetic products destined to improve responses of individual parts of the body to convulsion-related diseases.

## Patentansprüche

1. Verbindung der allgemeinen Formel I, 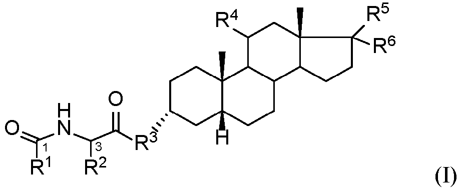 wobei
- R¹ ist Methyl, und dann R² ein Wasserstoffatom oder lineares oder verzweigtes C 1-C4-Alkyl ist,
oder
R¹ und R² bilden zusammen eine Gruppe -(CH₂)ₚ-, wobei p=2 oder 3 ist, die zusammen mit den Kohlenstoffatomen 1 und 3 und mit dem Stickstoffatom der allgemeinen Formel I einen fünf- oder sechsgliedrigen Ring bildet,
- R³ ist -O-(CH₂)ₙ -Oₘ-, wobei n = 0, 1 oder 2 und m = 0 oder 1,
- R⁴ ist ein Wasserstoffatom oder eine Hydroxylgruppe,
- R⁵ ist ein Wasserstoffatom, und dann ist R⁶ ausgewählt aus einer Gruppe bestehend aus Wasserstoffatom, Acetylgruppe, Cyanogruppe, C₁-C₂-Cyanoalkylgruppe, 1,1-Difluorethylgruppe und linearem oder verzweigtem C₁-C₄-Alkyl,
oder R⁵ und R⁶ bilden zusammen eine Struktur, ausgewählt aus einer Gruppe bestehend aus C₁-C₂-Alkyliden, Cyanomethylengruppe, Sauerstoffatom, zwei Fluoratomen.

2. Verbindung der allgemeinen Formel I nach Anspruch 1, ausgewählt aus einer Gruppe bestehend aus:
(3R5R,8R,9S,10S,13S,14S,17S)-17-Acetyl-10,13-dimethylhexadecahydro-1H-cyclopenta[a]fenantren-3-yl 5-oxopyrrolidin-2-carboxylat (1),
(3R5R,8R,9S,10S,13S,14S,17S)-17-Acetyl-10,13-dimethylhexadecahydro-1H-cyclopenta[a]fenantren-3-yl 6-oxopiperidin-2-carboxylat (2)
(3R,5R,8R,9S,10S,13S,14S,17S)-17-Cyano-10,13-dimethylhexadecahydro-1H-cyclopenta[a]fenantren-3-yl 5-oxopyrrolidin-2-carboxylat (3),
(3R,5R,8S,9S,10S,11R,13S,14S,17S)-17-Acetyl-11-hydroxy-10,13-dimethylhexadecahydro-1H-cyclopenta[a]fenantren-3-yl 5-oxopyrrolidin-2-carboxylat (4),
(3R,SR,8S,9S, 10S, 13S, 14S)-10, 13-Dimethylhexadecahydro-1H-cyclopenta[a]fenantren-3-yl 5-oxopyrrolidin-2-carboxylat (5),
(3R,5R,8R,9S,10S,13S,14S)-10,13-Dimethyl-17-oxohexadecahydro-1H-cyclopenta[a]fenantren-3-yl 5-oxopyrrolidin-2-carboxylat (6),
(3R,SR,8S,9S,10S,13S,14S)-10,13-Dimethylhexadecahydro-1H-cyclopenta[a]fenantren-3-yl 6-oxopiperidin-2-carboxylat (7),
(3R,5R,8R,9S,10S,13S,14S,17S)-17-Cyano-10,13-dimethylhexadecahydro-1H-cyclopenta[a]fenantren-3-yl 6-oxopiperidin-2-carboxylat (8),
(3R,5R,8R,9S,10S,13S,14S,17S)-17-Acetyl-10,13-dimethylhexadecahydro-1H-cyclopenta[a]fenantren-3-yl-acetylglycinat (9),
(3R,5R,8R,9S,10S,13S,14S,17S)-17-Acetyl-10,13-dimethylhexadecahydro-1H-cyclopenta[a]fenantren-3-yl-acetylleucinat (10),
(3R,5R,8S,9S,10S,13R,14S,17S)-10,13,17-Trimethylhexadecahydro-1H-cyclopenta[a]fenantren-3-yl-5-oxopyrrolidin-2-carboxylat (11),
(3R,5R,8R,9S,10S,13S,14S,Z)-17-Ethyliden-10,13-dimethylhexadecahydro-1H-cyclopenta[a]fenantren-3-yl 5-oxopyrrolidin-2-carboxylat (12),
2-(((3R,5R,8S,9S,10S,13S,14S)-10,13-Dimethylhexadecahydro-1H-cyclopenta[a]fenantren-3-yl)oxy)ethyl 5-oxopyrrolidin-2-carboxylat (13),
2-(((3R,5R,8S,9S,10S,13S,14S)-10,13-Dimethylhexadecahydro-1H-cyclopenta[a]fenantren-3-yl)oxy)ethyl 6-oxopiperidin-2-carboxylat (14),
2-((3R,5R,8R,9S,10S,13S,14S,17S)-17-Acetyl-10,13-dimethylhexadecahydro-1H-cyclopenta[a]fenantren-3-yl)ethyl 5-oxopyrrolidin-2-carboxylat (16),
2-((3R,5R,8R,9S,10S,13S,14S,17S)-17-Acetyl-10,13-dimethylhexadecahydro-1H-cyclopenta[a]fenantren-3-yl)ethyl 6-oxopiperidin-2-carboxylat (17).

3. Verbindung der allgemeinen Formel I nach Anspruch 1 oder 2 zur Verwendung als Arzneimittel.

4. Verbindung der allgemeinen Formel I nach Anspruch 1 oder 2 zur Verwendung bei der Behandlung von Epilepsie oder mit Krampfanfällen verbundenen Zuständen, wie Anfälle in Verbindung mit Hypoxie; Anfälle in Verbindung mit traumatischen Hirnschäden; Anfälle in Verbindung mit Intoxikation; pathologische Veränderungen durch Übererregung.

5. Verbindung der allgemeinen Formel I nach Anspruch 1 von 2 zur Verwendung bei der Behandlung von Zuständen, die mit Epilepsie einhergehen, wie affektive Störungen, Depression, posttraumatische Belastungsstörung (PTSD) und stressbedingte Erkrankungen, Angstzustände, Schizophrenie und psychotische Störungen, damit verbundene ischämische ZNS-Schäden, neurodegenerative Veränderungen und Erkrankungen, Multiple Sklerose.

6. Verwendung der Verbindung der allgemeinen Formel I nach Anspruch 1 oder 2 bei der Herstellung eines Arzneimittels zur Verwendung bei der Behandlung von Epilepsie oder mit Epilepsie verbundenen Komorbiditäten oder mit Krämpfen verbundenen Zuständen, wie Anfällen in Verbindung mit Hypoxie; Anfällen in Verbindung mit traumatischen Hirnschäden; Anfällen in Verbindung mit Intoxikation; pathologischen Veränderungen, die durch Übererregung verursacht werden; oder bei der Behandlung von Zuständen, die mit Epilepsie einhergehen, wie affektiven Störungen, Depressionen, posttraumatischen Belastungsstörungen (PTBS) und stressbedingten Erkrankungen, Angstzuständen, Schizophrenie und psychotischen Störungen, damit verbundenen ischämischen ZNS-Schäden, neurodegenerativen Veränderungen und Störungen, Multipler Sklerose.

7. Pharmazeutische Zusammensetzung, **dadurch gekennzeichnet, dass** sie mindestens eine Verbindung der allgemeinen Formel I nach Anspruch 1 oder 2 als Wirkstoff und mindestens einen pharmazeutisch verträglichen Träger enthält.

8. Pharmazeutische Zusammensetzung nach Anspruch 7 zur Behandlung von Epilepsie und damit verbundenen Komorbiditäten oder mit Krämpfen verbundenen Zuständen, wie Anfällen in Verbindung mit Hypoxie; Anfällen in Verbindung mit traumatischen Hirnschäden; Anfällen in Verbindung mit Intoxikation; pathologischen Veränderungen durch Übererregung; oder zur Behandlung von Begleitzuständen von Epilepsie, wie affektiven Störungen, Depression, posttraumatischer Belastungsstörung (PTSD) und stressbedingten Erkrankungen, Angstzuständen, Schizophrenie und psychotischen Störungen, damit verbundenen ischämischen ZNS-Schäden, neurodegenerativen Veränderungen und Erkrankungen, Multipler Sklerose.

9. Verbindung nach Anspruch 1 oder 2 zur Verwendung in einem Verfahren zur Behandlung von Epilepsie und damit verbundenen Komorbiditäten oder anderen mit Krämpfen verbundenen Zuständen, wie Anfällen in Verbindung mit Hypoxie; Anfällen in Verbindung mit traumatischen Hirnschäden; Anfällen in Verbindung mit Intoxikation; pathologischen Veränderungen durch Übererregung; oder zur Behandlung von Zuständen, die mit Epilepsie einhergehen können, wie affektive Störungen, Depression, posttraumatische Belastungsstörung (PTSD) und stressbedingte Erkrankungen, Angstzustände, Schizophrenie und psychotische Störungen, damit verbundene ischämische ZNS-Schäden, neurodegenerative Veränderungen und Erkrankungen, Multiple Sklerose, wobei das Verfahren den Schritt der Verabreichung von mindestens einer Verbindung der allgemeinen Formel I nach Anspruch 1 an einen Patienten umfasst, der einer solchen Behandlung bedarf.

10. Verbindung der allgemeinen Formel I nach Anspruch 1 oder 2 zur Verwendung als Wirkstoff oder Hilfsstoff in Nahrungsergänzungsmitteln oder kosmetischen Produkten, die dazu bestimmt sind, die Reaktionen einzelner Körperteile auf krampfbedingte Erkrankungen zu verbessern.

## Revendications

1. Composé de formule générale I, 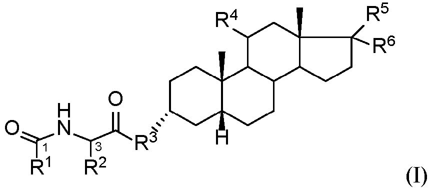 où
- R¹ représente un méthyle, puis R² est un atome d'hydrogène ou un alkyle en C₁-C₄ linéaire ou ramifié,
ou
R¹ et R² forment ensemble un groupe -(CH₂)ₚ-, où p=2 ou 3, qui forme un cycle à cinq ou six chaînons avec les atomes de carbone 1 et 3 et avec l'atome d'azote de formule générale I,
- R³ est -O-(CH₂)ₙ-Oₘ-, où n = 0, 1 ou 2 et m = 0 ou 1,
- R⁴ est un atome d'hydrogène ou un groupe hydroxyle,
- R⁵ est un atome d'hydrogène, puis R⁶ est choisi dans un groupe constitué d'un atome d'hydrogène, d'un groupe acétyle, d'un groupe cyano, d'un groupe cyanoalkyle en C₁-C₂, d'un groupe 1,1-difluoroéthyle et d'un alkyle en C₁-C₄ linéaire ou ramifié,
ou R⁵ et R⁶ forment ensemble une structure choisie dans un groupe constitué d'alkylidène en C₁-C₂, d'un groupe cyanométhylène, d'un atome d'oxygène, de deux atomes de fluor.

2. Composé de formule générale I selon la revendication 1, choisi dans un groupe constitué de:
(3R,5R,8R,9S,10S,13S,14S,17S)-17-acétyl-10,13-diméthylhexadécahydro-1H-cyclopenta[a]phénantrène-3-yle 5-oxopyrrolidine-2-carboxylate (1),
(3R,5R,8R,9S,10S,13S,14S,17S)-17-acétyl-10,13-diméthylhexadécahydro-1H-cyclopenta[a]phénantrène-3-yle 6-oxopipéridine-2-carboxylate (2)
(3R,5R,8R,9S,10S,13S,14S,17S)-17-cyano-10,13-diméthylhexadécahydro-1H-cyclopenta[a]phénanthrène-3-yle 5-oxopyrrolidine-2-carboxylate (3),
(3R,5R,8S,9S,10S,11R,13S,14S,17S)-17-acétyl-11-hydroxy-10,13-diméthylhexadécahydro-1H-cyclopenta[a]phénanthrène-3-yle 5-oxopyrrolidine-2-carboxylate (4),
(3R,5R,8S,9S,10S,13S,14S)-10,13-diméthylhexadécahydro-1H-cyclopenta[a]phénanthrène-3-yle 5-oxopyrrolidine-2-carboxylate (5),
(3R,5R,8R,9S,10S,13S,14S)-10,13-diméthyl-17-oxohexadécahydro-1H-cyclopenta[a]phénanthrène-3-yle 5-oxopyrrolidine-2-carboxylate (6),
(3R,5R,8S,9S,10S,13S,14S)-10,13-diméthylhexadécahydro-1H-cyclopenta[a]phénanthrène-3-yle 6-oxopipéridine-2-carboxylate (7),
(3R,5R,8R,9S,10S,13S,14S,17S)-17-cyano-10,13-diméthylhexadécahydro-1H-cyclopenta[a]phénanthrène-3-yle 6-oxopipéridine-2-carboxylate (8),
(3R,5R,8R,9S,10S,13S,14S,17S)-17-acétyl-10,13-diméthylhexadécahydro-1H-cyclopenta[a]phénanthrène-3-yle acétylglycinate (9),
(3R,5R,8R,9S,10S,13S,14S,17S)-17-acétyl-10,13-diméthylhexadécahydro-1H-cyclopenta[a]phénanthrène-3-yle acétylleucinate (10),
(3R,5R,8S,9S,10S,13R,14S,17S)-10,13,17-triméthylhexadécahydro-1H-cyclopenta[a]phénanthrène-3-yle 5-oxopyrrolidine-2-carboxylate (11),
(3R,5R,8R,9S,10S,13S,14S,Z)-17-éthylidène-10,13-diméthylhexadécahydro-1H-cyclopenta[a]phénanthrène-3-yl 5-oxopyrrolidine-2-carboxylate (12),
2-(((3R,5R,8S,9S,10S,13S,14S)-10,13-diméthylhexadécahydro-1H-cyclopenta[a]phénanthrène-3-yl)oxy)éthyl 5-oxopyrrolidine-2-carboxylate (13),
2-(((3R,5R,8S,9S,10S,13S,14S)-10,13-diméthylhexadécahydro-1H-cyclopenta[a]phénanthrène-3-yl)oxy)éthyl 6-oxopipéridine-2-carboxylate (14),
2-((3R,5R,8R,9S,10S,13S,14S,17S)-17-acétyl-10,13-diméthylhexadécahydro-1H-cyclopenta[a]phénanthrène-3-yl)éthyl 5-oxopyrrolidine-2-carboxylate (16),
2-((3R,5R,8R,9S,10S,13S,14S,17S)-17-acétyl-10,13-diméthylhexadécahydro-1H-cyclopenta[a]phénanthrène-3-yl)éthyl 6-oxopipéridine-2-carboxylate (17).

3. Composé de formule générale I selon la revendication 1 ou 2 pour utilisation comme médicament.

4. Composé de formule générale I selon la revendication 1 ou 2 pour utilisation dans le traitement de l'épilepsie ou d'affections associées à des convulsions, telles que les crises associées à l'hypoxie; les crises associées à des lésions cérébrales traumatiques; les crises associées à une intoxication; les changements pathologiques causés par une hyperexcitation.

5. Composé de formule générale I selon la revendication 1 ou 2 pour utilisation dans le traitement d'affections accompagnant l'épilepsie, telles que les troubles affectifs, la dépression, le trouble de stress post-traumatique (TSPT) et les maladies liées au stress, l'anxiété, la schizophrénie et les troubles psychotiques, les lésions ischémiques du SNC associées, les changements et troubles neurodégénératifs, la sclérose en plaques.

6. Utilisation du composé de formule générale I selon la revendication 1 ou 2 dans la fabrication d'un médicament pour utilisation dans le traitement de l'épilepsie ou de comorbidités associées à l'épilepsie ou d'affections associées à des convulsions, telles que des crises associées à une hypoxie; des crises associées à des lésions cérébrales traumatiques; des crises associées à une intoxication; des changements pathologiques causés par une hyperexcitation; ou dans le traitement d'affections accompagnant l'épilepsie, telles que des troubles affectifs, la dépression, le trouble de stress post-traumatique (TSPT) et les maladies liées au stress, l'anxiété, la schizophrénie et les troubles psychotiques, les lésions ischémiques du SNC associées, les changements et troubles neurodégénératifs, la sclérose en plaques.

7. Composition pharmaceutique, **caractérisée en ce qu'**elle contient, en tant que principe actif, au moins un composé de formule générale I selon la revendication 1 ou 2, et au moins un excipient pharmaceutiquement acceptable.

8. Composition pharmaceutique selon la revendication 7 pour le traitement de l'épilepsie et des comorbidités associées à l'épilepsie ou d'affections associées à des convulsions, telles que les crises associées à l'hypoxie; les crises associées à des lésions cérébrales traumatiques; les crises associées à une intoxication; les changements pathologiques causés par une hyperexcitation; ou dans le traitement des états accompagnant l'épilepsie, tels que les troubles affectifs, la dépression, le trouble de stress post-traumatique (TSPT) et les maladies liées au stress, l'anxiété, la schizophrénie et les troubles psychotiques, les lésions ischémiques du SNC associées, les changements et troubles neurodégénératifs, la sclérose en plaques.

9. Composé selon la revendication 1 ou 2 pour utilisation dans une méthode de traitement de l'épilepsie et des comorbidités qui lui sont associées ou d'affections associées à des convulsions, telles que les crises associées à l'hypoxie; les crises associées à des lésions cérébrales traumatiques; les crises associées à une intoxication; les changements pathologiques causés par une hyperexcitation; ou pour le traitement d'affections accompagnant l'épilepsie, telles que les troubles affectifs, la dépression, le trouble de stress post-traumatique (TSPT) et les maladies liées au stress, l'anxiété, la schizophrénie et les troubles psychotiques, les lésions ischémiques du système nerveux central associées, les changements et troubles neurodégénératifs, la sclérose en plaques, ledit procédé comprenant l'étape d'administration d'au moins un composé de formule générale I selon la revendication 1 à un patient nécessitant un tel traitement.

10. Composé de formule générale I selon la revendication 1 ou 2 pour utilisation comme principe actif ou principe auxiliaire dans des compléments alimentaires ou des produits cosmétiques destinés à améliorer les réponses de parties individuelles du corps aux maladies liées aux convulsions.
